# EUROPEAN PATENT APPLICATION

(11) **EP 1 126 029 A1**
(43) Date of publication of application: **22.08.2001**
(21) Application number: 99951094.4
(22) Date of filing: 27.10.1999
(51) Int. Cl.: C12N 15/12, C07K 14/705, C07K 16/28, C12Q 1/02, C12Q 1/68, C12N 5/10, C12N 1/21, C12P 21/02, C07H 21/04, A61K 38/00

(54) **NOVEL G PROTEIN-COUPLED RECEPTOR PROTEINS AND DNAS THEREOF**

(30) Priority: 28.10.1998 JP 30654398; 28.01.1999 JP 2035699
(71) Applicant: Takeda Chemical Industries, Ltd., Osaka-shi, Osaka 541-0045 (JP)
(72) Inventor: WATANABE, Takuya, Tsukuba-shi, Ibaraki 305-0821 (JP); MATSUMOTO, Yoshio, Ikeda-shi, Osaka 563-0029 (JP); TERAO, Yasuko, Tsukuba-shi, Ibaraki 305-0034 (JP); SHINTANI, Yasushi, Tsukuba-shi, Ibaraki 305-0821 (JP)
(74) Representative: Keller, Günter, Dr.
(86) International application number: JP9905938
(87) International publication number: WO0024891

(57) **Abstract**

The present invention relates to rat peripheral brainstem-derived and human fetus-derived G protein coupled receptor protein, a partial peptide thereof or a salt thereof, and nucleic acid encoding the receptor protein, and a derivetive thereof.

The present rat peripheral brainstem-derived and human fetus-derived G protein coupled receptor proteins or nucleic acid encoding the same and a derivative thereof can be used for determining a ligand (agonist) for the present receptor protein, an agent for preventing and/or treating diseases associated with the deficient functions of the present G protein coupled receptor protein, a gene therapeutic, and a method for screening a compound which alters an amount of the present receptor protein or a partial peptide thereof.

## Description

### Technical field

The present invention relates to a rat peripheral brainstem-derived and human fetus-derived novel G protein coupled receptor protein or a salt thereof and a DNA encoding it.

### Background Art

Physiologically active substances such as many hormones and neurotransmitters regulate the function of the living body through specific receptor proteins present in a cell membrane. Since many of these receptor proteins conduct intracellular signal transmission through activation of coupled guanine nucleotide-binding protein (hereinafter abbreviated as G protein in some cases) and have the common structure having 7 transmembrane regions, they are called collectively as G protein coupled receptor protein or 7 times transmembrane type receptor protein (7 TMR).

The G protein coupled receptor protein is present on the surfaces of the cells and various functional cells of organs of the living body and plays on a physiologically important role as a target for a molecule which regulates the functions of those cells and organs, for example, hormones, neurotransmitters and physiologically active substances. A receptor transmits a signal into a cell through binding with a physiologically active substance, and a variety of reactions such as activation and inhibition of a cell are induced by this signal.

Elucidation of the relationship of a substance which regulates the complex function of cells and organs of a variety of living bodies and a specific receptor therefor, in particular, a G protein coupled receptor protein elucidates the function of cells and organs of a variety of living bodies, and provides a very important means for developing drugs which are closely associated with the functions.

For example, in a variety of organs of the living body, the physiological regulation is conducted under regulation by many hormones, hormone-like substances, neurotransmitters or physiologically active substances. In particular, physiologically active substances are present in various sites in the living body, and regulate their physiological function through receptor proteins, each corresponding thereto. There are still unknown hormones and neurotransmitters and other physiologically active substances in the living body, many of which have not been reported yet regarding the structures of these receptor proteins. Further, whether or not a subtype is present is not known in many of the known receptor proteins.

Revelation of the relationship between a substance which regulates the complex function in the living body and a specific receptor protein therefor is a very important means for developing medicines. In addition, in order to effectively screen an agonist and an antagonist for receptor proteins and develop medicines, it is necessary to elucidate the function of a gene of a receptor protein expressed in the living body and express it in a suitable expression system.

Recently, as a means for analyzing a gene expressed in the living body, studies of analyzing randomly a sequence of a cDNA are actively conducted, and the thus obtained sequences of cDNA fragments are registered in database as Expressed Sequence Tag (EST) are published. However, many ESTs contain only sequence information and it is difficult to presume their functions.

Previously, a substance which inhibits binding of a G protein coupled receptor and a physiologically active substance (that is, ligand) and a substance which causes the same signal transmission as that for a physiologically active substance (that is, ligand) by binding have been utilized as a medicine for regulating the functions of the living body as a specific antagonist or agonist for these receptors. Therefore, new finding of a G protein coupled receptor protein which is not only important in the physiological manifestation in the living body but also as a target for drug development, and cloning of the gene (for example, cDNA) are a very important means upon finding of a specific ligand, an agonist and an antagonist for a novel G protein coupled receptor protein.

However, not all of G protein coupled receptors have been found and, even at present, there are still many unknown G protein coupled receptors, and so called orphan receptors for which corresponding ligands have not been identified, and there are desired search of new G protein coupled receptors and elusidation of their functions.

A G protein coupled receptor is useful for searching a new physiologically active substance (that is, ligand) using its signal transmission action as an index, or searching an agonist or antagonist for the receptor. On the other hand, even when a physiological ligand has not been found, an agonist or an antagonist to the receptor can be prepared by analyzing the physiological action of the receptor from an inactivation experiment of the receptor (knockout animal). A ligand, an agonist or an antagonist for these receptors can be expected to be utilized as an agent for preventing/treating or diagnosing diseases associated with the insufficient function of a G protein coupled receptor.

Further, there are many cases where reduction or exasperation of the function of the receptor in the living body based on a gene mutation of a G protein coupled receptor is a cause for some diseases. In this case, there can be applied to gene therapy by not only administration of an antagonist or an agonist for receptor but also introduction of the receptor gene into the living body (or a particular organ) and introduction of an antisense nucleic acid for the receptor gene. In this case, a base sequence of the receptor is essential information for examining the presence or absence of deletion or mutation on a gene and the receptor gene can be applied to an agent for preventing/treating or diagnosing diseases associated with the insufficient function of the receptor.

### Disclosure of Invention

The present invention provides a novel G protein coupled receptor protein which is useful as described above. That is, the present invention provides a novel G protein coupled receptor protein or a partial peptide thereof or a salt thereof, a polynucleotide (DNA, RNA and their derivatives) comprising a polynucleotide (DNA, RNA and their derivatives) encoding the G protein coupled receptor protein or a partial peptide thereof, a recombinant vector comprising polynucleotide, a transformant harboring the recombinant vector, a process for producing the G protein coupled receptor protein or a salt thereof, an antibody to the G protein coupled receptor protein or a partial peptide thereof or a salt thereof, a compound which alters an amount of the G protein coupled receptor protein to be expressed, a method for determining a ligand for the G protein coupled receptor, a method for screening a compound (antagonist, agonist) or a salt thereof which alters binding of a ligand with the G protein coupled receptor protein, a kit for the screening, a compound (antagonist, agonist) which alters binding of a ligand obtained by using the screening method or screening kit and the G protein coupled receptor protein or the salt thereof, and a medicine comprising a compound (antagonist, agonist) or a salt thereof which alters binding of a ligand with the G protein coupled receptor protein and a compound which alters an amount of the G protein coupled receptor protein to be expressed.

The present inventors studied intensively and, as a result, we isolated a cDNA encoding a novel G protein coupled receptor protein derived from rat peripheral brainstem and human fetus based on EST information made by a degenerated PCR method, and succeeded in analyzing its entire base sequence. And when this base sequence was translated into an amino acid sequence, the first to seventh transmembrane regions were confirmed on hydrophobic plot, and proteins encoding by these cDNAs were confirmed to be 7 times transmembrane type G protein coupled receptor protein. The present inventors further studied on these findings and, which resulted in completion of the present invention.

That is, the present invention provides:
(1) a G protein coupled receptor protein which comprises an amino acid sequence identical or substantially identical to an amino acid sequence represented by SEQ ID No:1 or a salt thereof,
(2) the G protein coupled receptor protein according to the above (1), wherein the amino acid sequence substantially identical to an amino acid sequence represented by SEQ ID No:1 is an amino acid sequence represented by SEQ ID No:2 or SEQ ID No:7 or a salt thereof,
(3) a partial peptide of the G protein coupled receptor protein according to above (1) or a salt thereof,
(4) a polynucleotide which comprises a polynucleotide having a base sequence encoding the G protein coupled receptor protein according to the above (1),
(5) the polynucleotide according to the above (4), which is a DNA,
(6) the polynucleotide according to the above (4), which has a base sequence represented by SEQ ID No:3, SEQ ID No:4 or SEQ ID No:8,
(7) a recombinant vector which comprises the polynucleotide according the above (4),
(8) a transformant transformed with the recombinant vector according to the above (7),
(9) a method for producing the G protein coupled receptor protein or a salt thereof, which comprises culturing a transformant according to the above (8) to produce the G protein coupled receptor protein according to the above (1),
(10) an antibody to the G protein coupled receptor protein according to the above (1) or the partial peptide according to the above (3) or a salt thereof,
(11) the antibody according to the above (10), which is a neutralizing antibody which inactivates signal transmission of the G protein coupled receptor protein according to the above (1),
(12) a diagnostic agent which comprises the antibody according to the above (10),
(13) a ligand for the G protein coupled receptor protein according to the above (1) or a salt thereof, which is obtainable by using the G protein coupled receptor protein according to the above (1) or the partial peptide according to the above (3) or a salt thereof,
(14) a medicine which comprises the ligand for the G protein coupled receptor protein according to the above (13),
(15) a method for determining a ligand for the G protein coupled receptor protein according to the above (1) or a salt thereof, which comprises using the G protein coupled receptor protein according to the above (1) or the partial peptide according to the above (3) or a salt thereof,
(16) a method for screening a compound which alters binding of a ligand with the G protein coupled receptor protein according to the above (1) or a salt thereof, which comprises using the G protein coupled receptor protein according to the above (1) or the partial peptide according to the above (3) or a salt thereof ,
(17) a kit for screening a compound which alters binding of a ligand with the G protein coupled receptor protein or a salt thereof according to the above (1), or a salt thereof, which comprises the G protein coupled receptor protein according to the above (1) or the partial peptide according to the above (3) or a salt thereof,
(18) a compound which alters binding of a ligand with the G protein coupled receptor protein or a salt thereof according to the above (1) or a salt thereof, which is obtainable by using the method for screening according to the above (16) or the kit for screening according to the above (17),
(19) a medicine which comprises a compound which alters binding of a ligand with the G protein coupled receptor protein or a salt thereof according to the above (1), or a salt thereof, which is obtainable by the method for screening according to the above (16) or the kit for screening according to the above (17),
(20) a polynucleotide which hybridizes with the polynucleotide according to the above (4) under the highly stringent conditions,
(21) a polynucleotide which comprises a base sequence complementary to the polynucleotide according to the above (4) or a part thereof,
(22) a method for quantitating a mRNA for the G protein coupled receptor protein according to the above (1), which comprises using the polynucleotide according to the above (4) or a part thereof,
(23) a method for quantitating the G protein coupled receptor protein according to the above (1), which comprises using the antibody according to the above (10),
(24) a method for diagnosing diseases associated with the function of the G protein coupled receptor according to the above (1), which comprises using the quantitating method according to the above (22) or the above (23),
(25) a method for screening a compound which alters an amount of the G protein coupled receptor according to the above (1) to be expressed, or a salt thereof, which comprises using the method for quantitating according to the above (22),
(26) a method for screening a compound which alters an amount of the G protein coupled receptor protein according to the above (1) or a salt thereof in a cell membrane, which comprises using the method for quantitating according to the above (23),
(27) a compound which alters an amount of the G protein coupled receptor protein according to the above (1) or a salt thereof, which is obtainable by using the method for screening according to the above (25),
(28) a compound which alters an amount of the G protein coupled receptor protein according to the above (1) or a salt thereof in a cell membrane, which is obtainable by using the method for screening according to the above (26), and so forth.

Further, the present invention provides:
(29) the G protein coupled receptor protein or a salt thereof according to the above (1), wherein the protein is a protein comprising (a) an amino acid sequence represented by SEQ ID No:1, an amino acid sequence in which 1 or 2 or more (preferably, around 1 to 30, more preferably around 1 to 9, more preferably a few (1 to 5) amino acids in an amino acid sequence represented by SEQ ID No:1 are deleted, (b) an amino acid sequence in which 1 or 2 or more (preferably, around 1 to 30, more preferably around 1 to 10, more preferably a few (1 to 5)) amino acids are added to an amino acid sequence represented by SEQ ID No:1, (c) an amino acid sequence in which 1 or 2 or more (preferably, around 1 to 30, more preferably around 1 to 10, more preferably a few (1 to 5)) amino acids in an amino acid sequence represented by SEQ ID No:1 are substituted with other amino acids, or (d) an amino acid of a combination of them,
(30) a method for determining the ligand according to the above (15), which comprises contacting the G protein coupled receptor protein or a salt thereof according to the above (1) or the partial peptide or a salt thereof according to the above (3), with a test compound,
(31) the method for determining a ligand according to the above (30), wherein a ligand is, for example, angiotensin, bombesin, canabinoide, cholecystokinin, glutamine, serotonin, melatonin, neuropeptide Y, opioid, purine, vasopressin, oxytocin, PACAP, secretin, glucagon, calcitonin, adrenomedulin, somatostatin, GHRH, CRF, ACTH, GRP, PTH, VIP (vasoactive intestinal polypeptide), somatostatin, dopamine, motilin, amyline, bradykinin, CGRP (calcitonin gene related peptide), leukotriene, pancreastatin, prostaglandin, thromboxane, adenosine, adrenaline, α and β-chemokine (for example, IL-8, GROα, GROβ, GROγ, NAP-2, ENA-78, PF4, IP10, GCP-2, MCP-1, HC14, MCP-3, I-309, MIP1α, MIP-1β, RANTES), endothelin, enterogastrin, histamine, neurotensin, TRH, pancreatic polypeptide or galanin,
(32) the method for screening according to the above (16), which comprises comparing (i) the case where the Gprotein coupled receptor protein or a salt thereof according to the above (1) or the partial peptide or a salt thereof according to the above (3) is contacted with a ligand, with (ii) the case where the G protein coupled receptor protein or a salt thereof according to the above (1) or the partial peptide or a salt thereof according to the above (3) is contacted with a ligand and a test compound,
(33) a method for screening a compound which alters binding of a ligand and the G protein coupled receptor protein or a salt thereof according to the above (1), or a salt thereof, which comprises measuring and comparing an amount of a labeled ligand for the G protein coupled receptor protein or a salt thereof according to the above (1) or the partial peptide or a salt thereof according to the above (3) in (i) the case where a labeled ligand is contacted with the G protein coupled receptor protein or a salt thereof according to the above (1) or the partial peptide or a salt thereof according to the above (3), and (ii) the case where a labeled ligand and a test compound are contacted with the G protein coupled receptor protein or a salt thereof according to the above (1) or the partial peptide or a salt thereof according to the above (3),
(34) a method for screening a compound which alters binding of a ligand with the G protein coupled receptor protein or a salt thereof according to the above (1), or a salt thereof, which comprises measuring and comparing an amount of a labeled ligand to a cell comprising the G protein coupled receptor protein according to the above (1) in (i) the case where a labeled ligand is contacted with the cell, and (ii) the case where a labeled ligand and a test compound are contacted with the cell,
(35) a method for screening a compound which alters binding of a ligand with the G protein coupled receptor protein or a salt thereof according to the above (1), or a salt thereof, which comprises measuring and comparing an amount of a labeled ligand to a membrane fraction of the cell comprising the G protein coupled receptor protein according to the above (1) in (i) the case where a labeled ligand is contacted with the membrane fraction of the cell, and (ii) case where a labeled ligand and a test compound are contacted with the membrane fraction of the cell,
(36) a method for screening a compound which alters binding of a ligand with the G protein coupled receptor protein or a salt thereof according to the above (1) or a salt thereof, which comprises measuring and comparing an amount of binding of a labeled ligand for the G protein coupled receptor protein in (i) where a labeled ligand is contacted with the G protein coupled receptor protein expressed on a cell membrane of a transformant according to the above (8) by culturing the transformant, and (ii) case where a labeled ligand and a test compound are contacted with the G protein coupled receptor protein expressed on a cell membrane of a transformant according to the above (8) by cul turfing the transformant,
(37) a method for screening a compound which alters binding of a ligand with the G protein coupled receptor protein or a salt thereof according to the above (1), or a salt thereof, which comprises measuring and comparing the cell stimulating activity via the G protein coupled receptor protein in (i) the case where a compound which activates the G protein coupled receptor protein or a salt thereof according to the above (1) with a cell comprising the G protein coupled receptor protein according to the above (1), and (ii) the case where a compound which activates the G protein coupled receptor protein or a salt thereof according to the above (1) and a test compound are contacted with a cell comprising the G protein coupled receptor protein according to the above (1),
(38) a method for screening a compound which alters binding of a ligand with the G protein coupled receptor protein or a salt thereof according to the above (1), or a salt thereof, which comprises measuring and comparing the cell stimulating activity via the G protein coupled receptor protein in the case where a compound which activates the G protein coupled receptor protein or a salt thereof according to the above (1) is contacted with the G protein coupled receptor protein expressed on a cell membrane of a transformant according to the above (8) by culturing the transformant, and the case where a compound which activates the G protein coupled receptor protein or a salt thereof according to the above (1) and a test compound are contacted with the G protein coupled receptor protein expressed on a cell membrane of a transformant according to the above (8) by culturing the transformant,
(39) the method for screening according to the above (37) or the above (38), wherein the compound which activates the G protein coupled receptor protein according to the above (1) is angiotensin, bombesin, canabinoide, cholecystokinin, glutamine, serotonin, melatonin, neuropeptide Y, opioid, purine, vasopressin, oxytocin, PACAP, secretin, glucagon, calcitonin, adrenomedulin, somatostatin, GHRH, CRF, ACTH, GRP, PTH, VIP (vasoactive intestinal polypeptide), somatostatin, dopamine, motilin, amyline, bradykinin, CGRP (calcitonin gene related peptide), leukotriene, pancreastatin, prostaglandin, thromboxane, adenosine, adrenaline, α and β-chemokine (for example, IL-8, GROα, GROβ, GROγ, NAP-2, ENA-78, PF4, IP10, GCP-2, MCP-1, HC14, MCP-3, I-309, MIP1α, MIP-1β, RANTES), endothelin, enterogastrin, histamine, neurotensin, TRH, pancreatic polypeptide or galanin,
(40) a compound which alters binding of a ligand with the G protein coupled receptor protein or a salt thereof according to the above (1), or a salt thereof, which is obtainable by the method for screening according to the above (32) to (39),
(41) a medicine which comprises a compound which alters binding of a ligand with the G protein coupled receptor protein or a salt thereof according to the above (1), or a salt thereof, which is obtainable by the method for screening according to the above (32) to (39),
(42) the kit for screening according to the above (17), which comprises a cell comprising the G protein coupled receptor protein according to the above (1),
(43) the kit for screening according to the above (17), which comprises a membrane fraction of a cell comprising the G protein coupled receptor protein according to the above (1),
(44) the kit for screening according to the above (17), which comprises the G protein coupled receptor protein expressed on a cell membrane of a transformant according to the above (8) by culturing the transformant,
(45) a compound which alters binding of a ligand with the G protein coupled receptor protein or a salt thereof according to the above (1), or a salt thereof, which is obtainable by the kit for screening according to the above (42) to (44),
(46) a medicine which comprises a compound which alters binding of a ligand with the G protein coupled receptor protein or a salt thereof according to the above (1), or a salt thereof, which is obtainable by the kit for screening according to the above (42) to (44),
(47) a method for quantitating the G protein coupled receptor protein or a salt thereof according to the above (1) or the partial peptide or a salt thereof according to the above (3), which comprises contacting the antibody according to the above (10), with the Gprotein coupled receptor protein according to the above (1) or the partial peptide according to the above (3) or a salt thereof,
(48) a method for quantitating the G protein coupled receptor protein according to the above (1) or the partial peptide according to the above (3) or a salt thereof in a test solution, which comprises competitively reacting the antibody according to the above (10), with a test solution and the labeled G protein coupled receptor protein according to the above (1) or the partial peptide according to the above (3) or a salt thereof, and measuring a proportion of the G protein coupled receptor protein according to the above (1) or the partial peptide according to the above (3) or a salt thereof bound to the antibody, and
(49) a method for quantitating the G protein coupled receptor protein according to the above (1) or the partial peptide according to the above (3) or a salt thereof in a test solution, which comprises reacting simultaneously or successively a test solution with the antibody according to the above (10) insolubilized on a carrier and the labeled antibody according to the above (10), and measuring the activity of a labeling agent on a insolubilized carrier.

### Brief Description of Drawings

FIG. 1 shows a base sequence of a DNA encoding a novel G protein coupled receptor protein rOT7T009C of the present invention derived from rat peripheral brainstem obtained in Example 1, and an amino acid sequence presumed therefrom (continues to FIG. 2).

FIG. 2 shows a base sequence of a DNA encoding a G protein coupled receptor protein rOT7T009C of the present invention derived from rat peripheral brainstem obtained in Example 1, and an amino acid sequence presumed therefrom (continues from FIG. 1 and continues to FIG. 3).

FIG. 3 shows a base sequence of a DNA encoding a G protein coupled receptor protein rOT7T009C of the present invention derived from rat peripheral brainstem obtained in Example 1, and an amino acid sequence presumed therefrom (continues from FIG. 2 and continues to FIG. 4).

FIG. 4 shows a base sequence of a DNA encoding a G protein coupled receptor protein rOT7T009C of the present invention derived from rat peripheral brainstem obtained in Example 1, and an amino acid sequence presumed therefrom (continues from FIG. 3).

FIG. 5 shows a base sequence of a DNA encoding a G protein coupled receptor protein rOT7T009T of the present invention derived from rat peripheral brainstem obtained in Example 1, and an amino acid sequence presumed therefrom (continues to FIG. 6).

FIG. 6 shows a base sequence of a DNA encoding a G protein coupled receptor protein rOT7T009T of the present invention derived from rat peripheral brainstem obtained in Example 1, and an amino acid sequence presumed therefrom (continues from FIG. 5 and continues to FIG. 7).

FIG. 7 shows a base sequence of a DNA encoding a G protein coupled receptor protein rOT7T009T of the present invention derived from rat peripheral brainstem obtained in Example 1, and an amino acid sequence presumed therefrom (continues from FIG. 6 and continues to FIG. 8).

FIG. 8 shows a base sequence of a DNA encoding a G protein coupled receptor protein rOT7T009T of the present invention derived from rat peripheral brainstem obtained in Example 1, and an amino acid sequence presumed therefrom (continues from FIG. 5 and continues from FIG. 7).

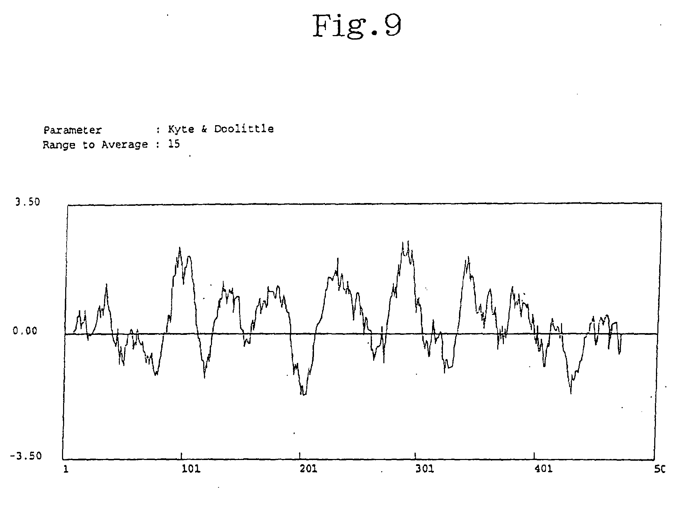

FIG. 9 shows a hydrophobic plot of the G protein coupled receptor proteins rOT7T009C and rOT7T009T of the present invention derived from rat peripheral brainstem, which was made based on amino acid sequences shown in FIG. 1 to FIG. 8.

FIG. 10 shows a base sequence of a DNA encoding a novel G protein coupled receptor protein hOT7T009 of the present invention derived from a human fetus obtained in Example 2, and an amino acid sequence presumed therefrom (continues to FIG. 11).

FIG. 11 shows a base sequence of a DNA encoding a G protein coupled receptor protein hOT7T009 of the present invention derived from a human fetus obtained in Example 2, and an amino acid sequence presumed therefrom (continues from FIG. 10 and continues to FIG. 12).

FIG. 12 shows a base sequence of a DNA encoding a G protein coupled receptor protein hOT7T009 of the present invention derived from a human fetus obtained in Example 2, and an amino acid sequence presumed therefrom (continues from FIG. 12).

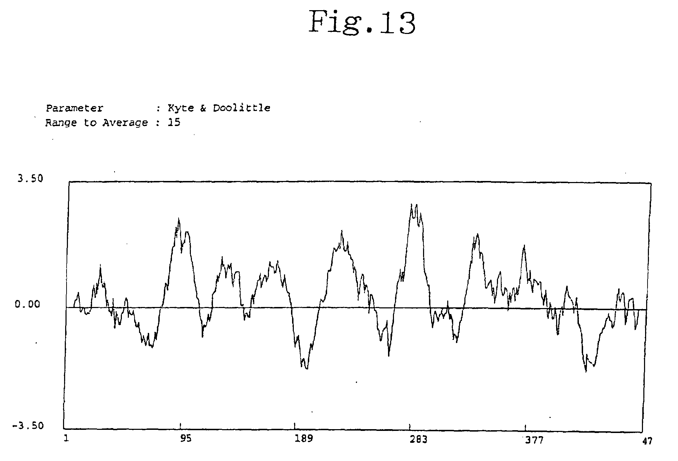

FIG. 13 shows a hydrophobic plot of the G protein coupled receptor protein hOT7T009 of the present invention derived from a human fetus, which was made based on amino acid sequences shown in FIG. 10 to FIG. 12.

### Best Mode for Carrying Out the Invention

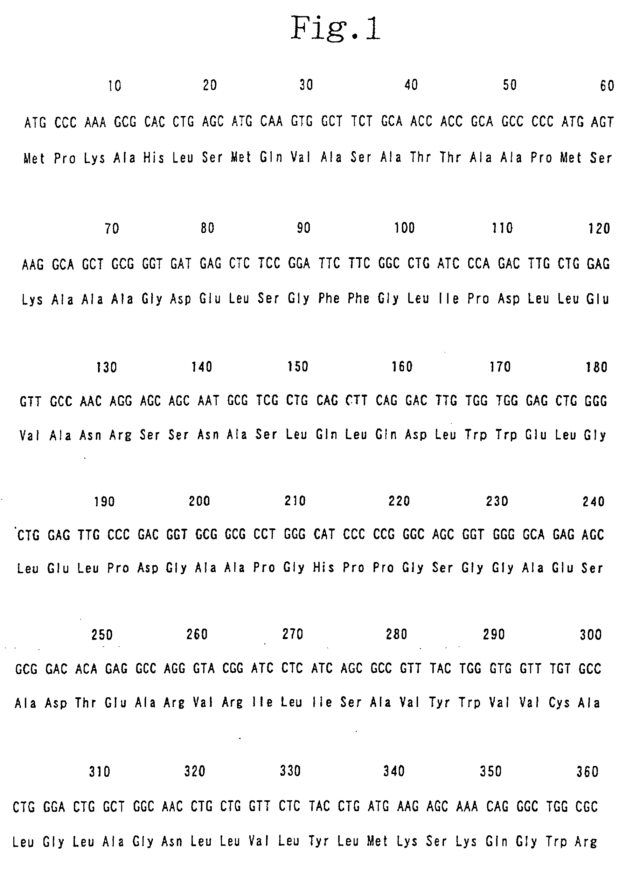

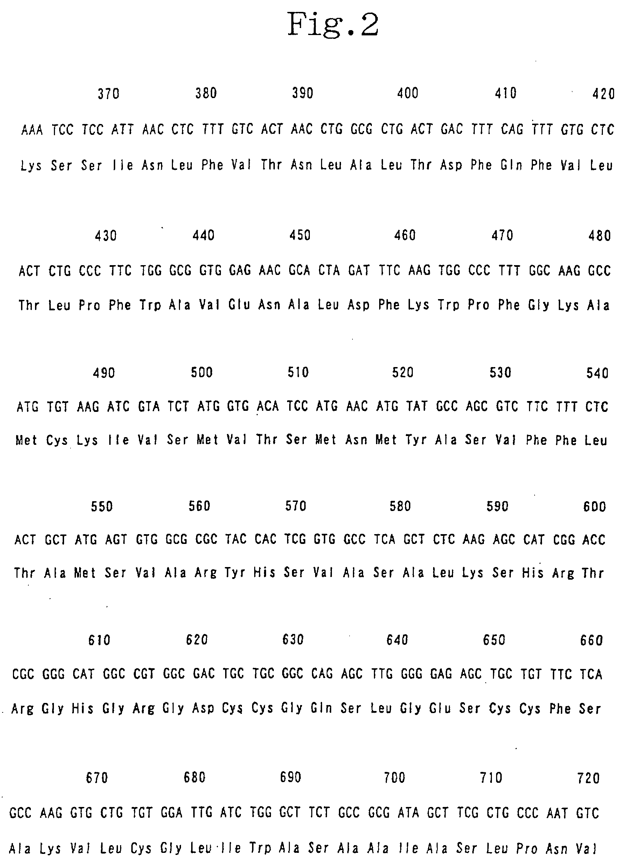

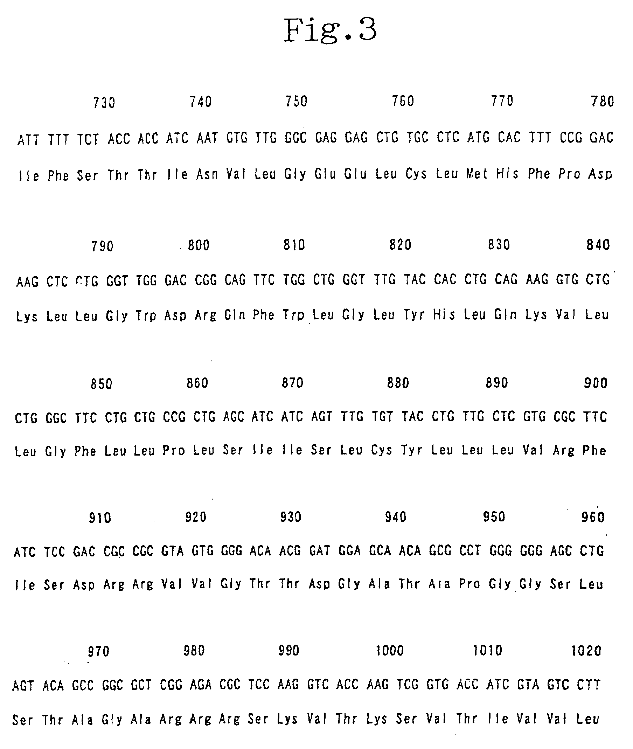

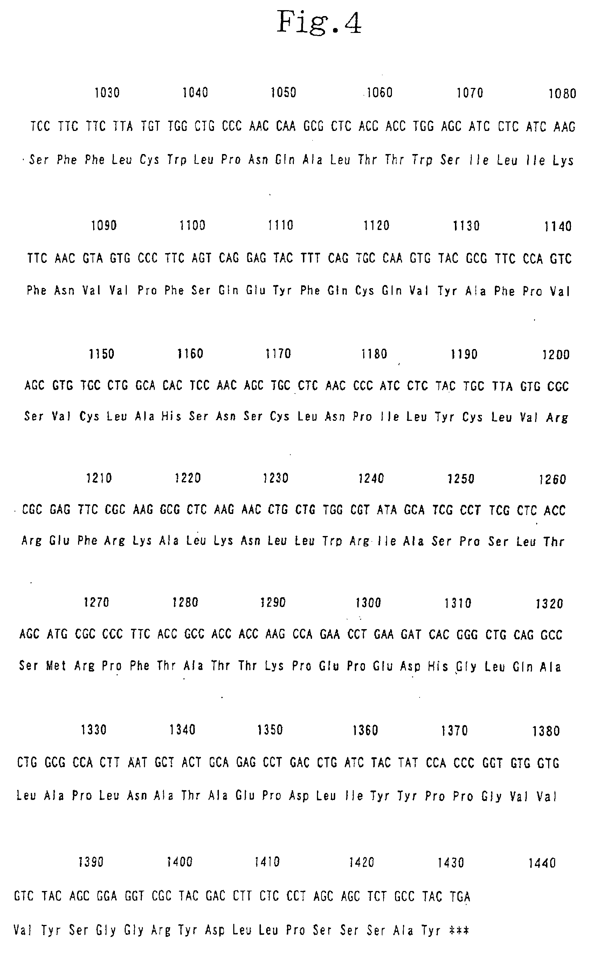

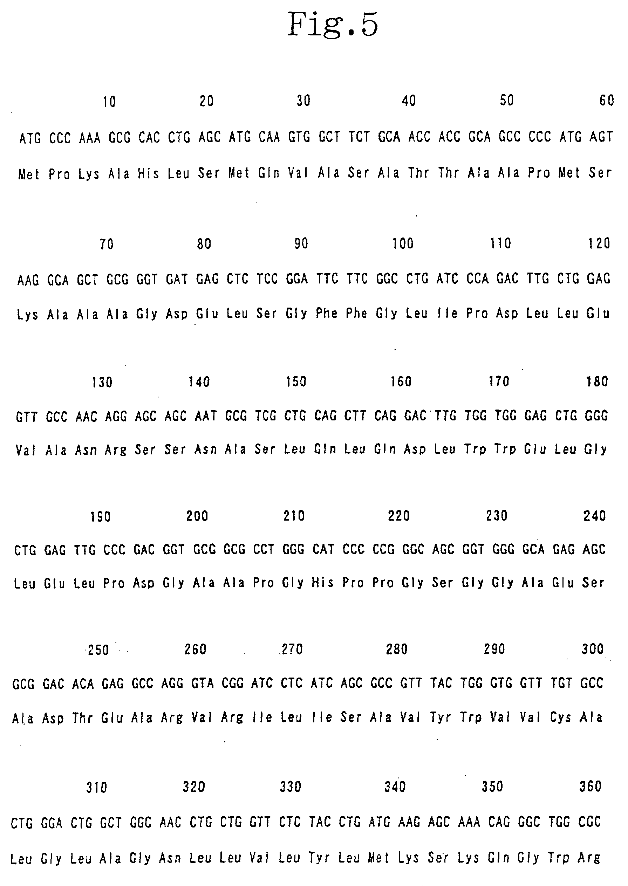

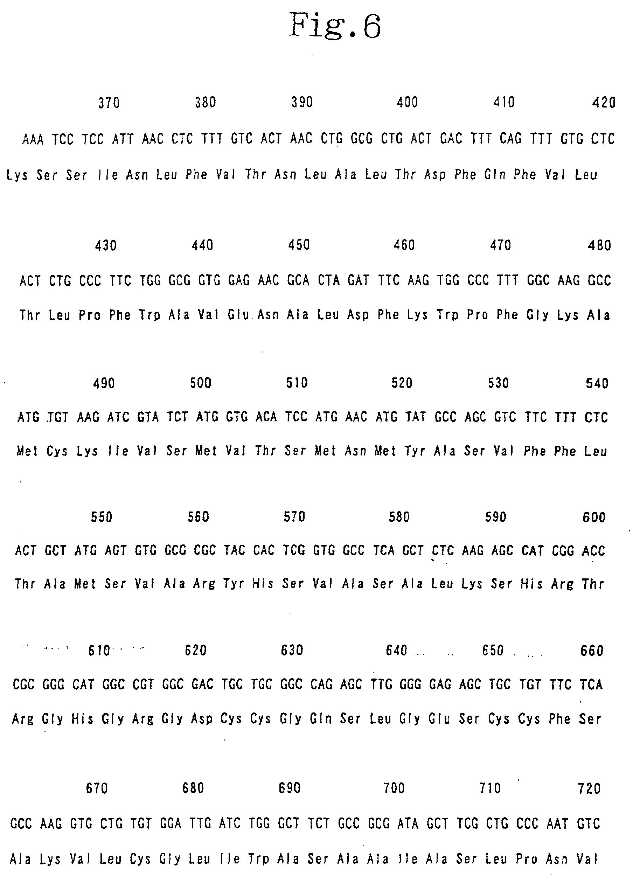

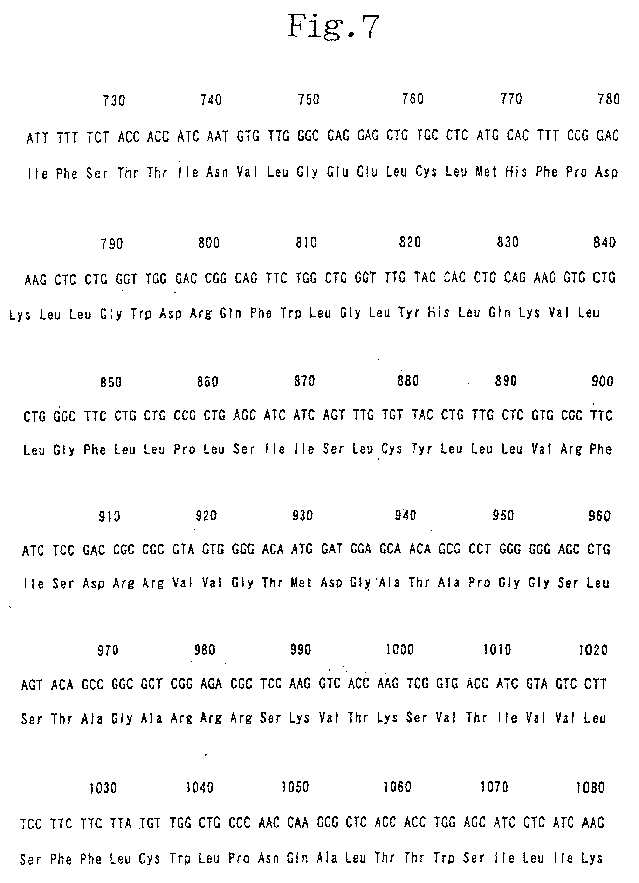

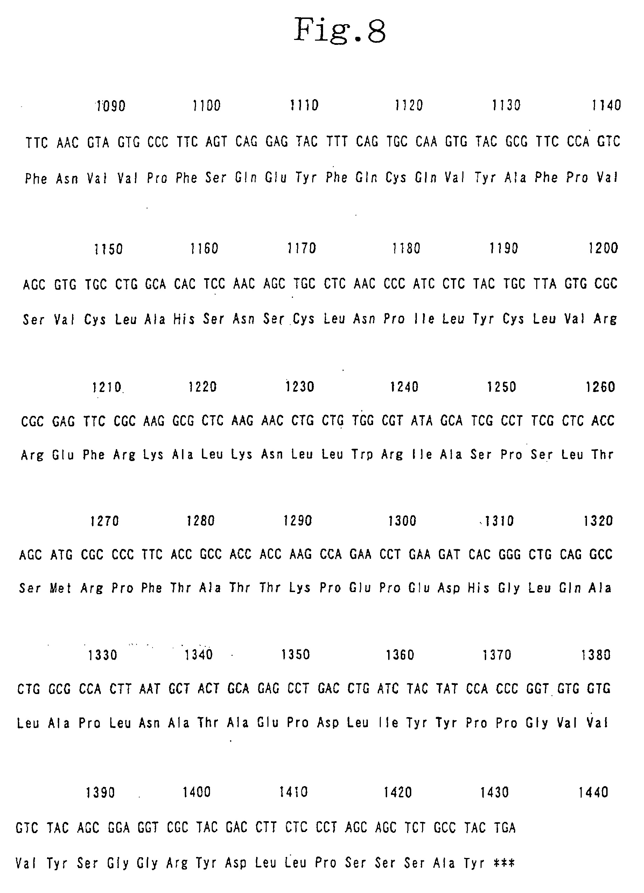

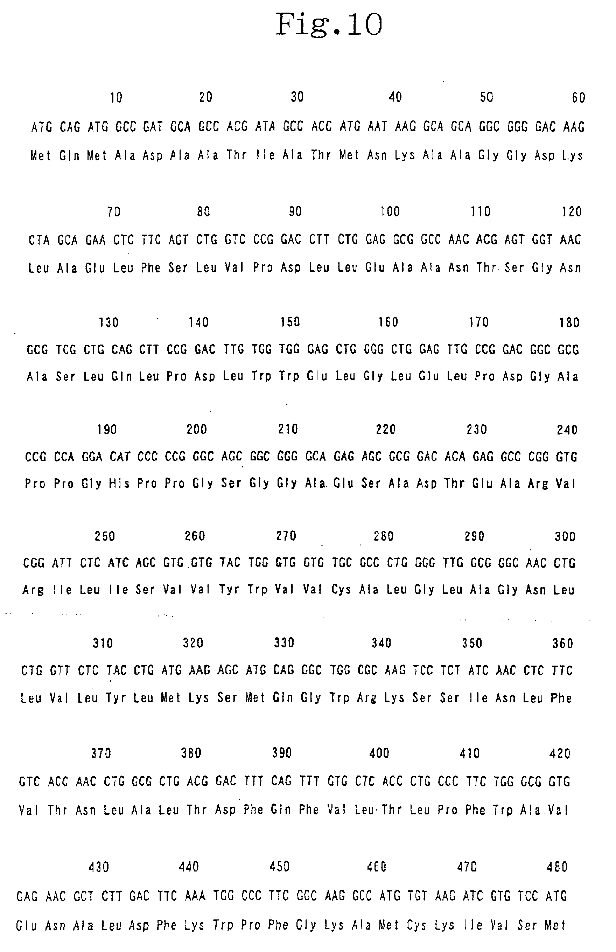

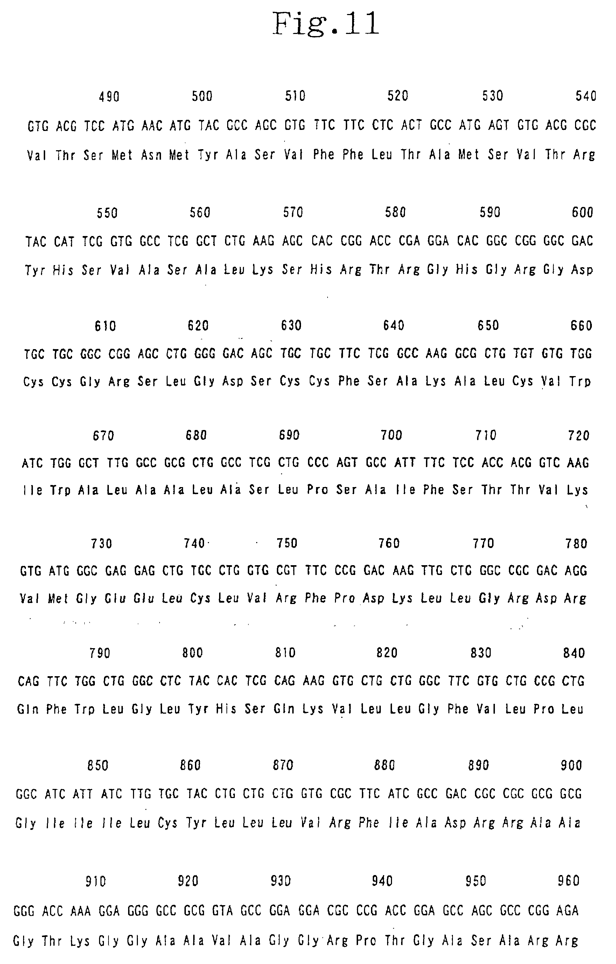

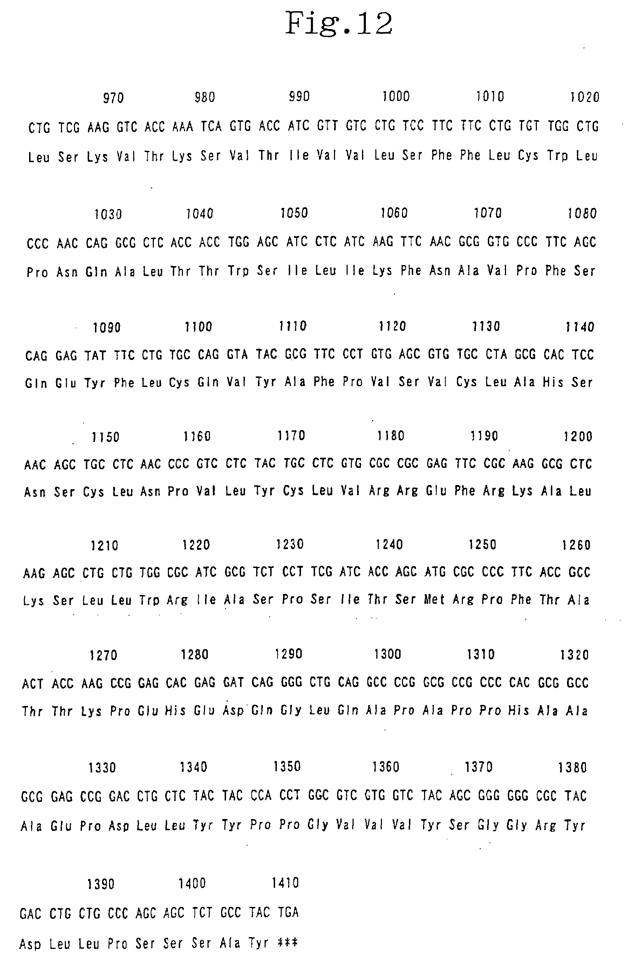

The present G protein coupled receptor protein (herinafter referred to as receptor protein in some cases) is a receptor protein comprising an amino acid sequence identical or substantially identical (for example, an amino acid sequence represented by SEQ ID No:2 (amino acid sequence in FIGS. 5-8) or an amino acid sequence represented by SEQ ID No:7 (amino acid sequence in FIGS.10-12)) to an amino acid sequence represented by SEQ ID No:1 (amino acid sequence in FIGs. 1 to 4).

The present receptor protein may be a protein derived from cells (e.g., hepatic cells, spleen cells, nerve cells, glia cells, pancreatic β-cells, bone marrow cells, mesangial cells, Langerhans' cells, epidermal cells, epithelial cells, endothelial cells, fibroblasts, fibrous cells, muscular cells, fat cells, immune cells (e.g., macrophage, T cell, B cell, natural killer cell, mast cell, neutrophils, basophils, eosinophils, monocyte), megakaryocyte, synovial membrane cells, soft-bone cells, bone cells, osteoblast, osteoclast, mammary gland cells or interstitial cells, or their precursor cells, stem cells or cancer cells) or any tissues having such cells, for example, brain and each site of brain (e.g., olfactory bulb, tonsil nuclei, cerebral basal bulb, hippocampus, thalamus, hypothalamus, cerebral cortex, medulla bulb, cerebellum), spinal cord, pituitary, stomach, pancreas, kidney, liver, gonads, thyroid glands, galls, bone marrow, adrenals, skin, muscles, lungs, digestive tracts (e.g., large and small intestines), blood vessels, heart, thymus, spleen, salivary glands, peripheral blood, prostate, testicles, ovary, placenta, uterus, bone, cartilage, joints, skeletal muscles from humans or warm-blooded animals (e.g., guinea pig, rat, mouse, chicken, rabbit, pig, sheep, cow, monkey etc.), as well as a recombinant protein or a synthetic protein.

Examples of an amino acid sequence substantially identical to an amino acid sequence represented by SEQ ID No: 1 include an amino acid sequence having about 50% or more, preferably about 70% or more, more preferably about 80% or more, more preferably about 90% or more, most preferably about 95% or more homology with an amino acid sequence represented by SEQ ID No:1. More particularly, there is an amino acid sequence represented by SEQ ID No:2 or SEQ ID No:7.

The protein of the present invention comprising an amino acid sequence substantially identical with the amino acid sequence represented by SEQ ID NO:1 is preferably, for example, a protein having an amino acid sequence substantially identical with the amino acid sequence represented by SEQ ID NO:1 and having substantially homogeneous properties with those of a protein having the amino acid sequence represented by SEQ ID NO: 1.

The substantially homogeneous properties indicate, for example include the ligand binding activity and the signal information transmission action. The terms "substantially homogeneous" mean that those activities are qualitatively homogeneous. Therefore, although it is preferable that the activities such as the ligand binding activity and the signal information transmission action are equal (for example, about 0.01 to 100-fold, preferably about 0.5 to 20-fold, more preferably about 0.5 to 2-fold), quantitative elements such as a degree of these activities and a molecular weight may be different.

Measurement of the ligand binding activity and the signal information transmission action can be according to the per se known method, for example, by the ligand determining method and screening method described later.

In addition, as the receptor protein of the present invention, a protein comprising (a) an amino acid sequence in which 1 or 2 or more (preferably, around 1 to 30, more preferably around 1 to 10, further preferably a few (1 to 5)) amino acids in an amino acid sequence represented by SEQ ID No: 1 are deleted, (b) an mino acid sequence in which 1 or 2 or more (preferably, around 1 to 30, more preferably around 1 to 10, further preferably a few (1 to 5)) amino acids are added to an amino acid sequence represented by SEQ ID No: 1, (c) an amino acid sequence in which 1 or 2 or more (preferably, around 1 to 30, more preferably around 1 to 10, further preferably a few (1 to 5)) amino acids in an amino acid sequence represented by SEQ ID No:1 are substituted with other amino acids, or (d) an amino acid of a combination of them, and the like is used.

In receptor proteins in the present specification, a left end is a N-terminal (amino terminal) and a right end is a C-terminal (carboxyl terminal) according to the convention of the peptide display. Although present proteins including a protein comprising an amino acid sequence represented by SEQ ID No:1 have usually carboxyl group (-COOH) or carboxylate (-COO⁻) as a C-terminal, they may have an amide (-CONH₂) or an ester (-COOR) as a C-terminal.

As R in ester, for example, C₁₋₆ alkyl group such as methyl, ethyl, n-propyl, isopropyl and n-butyl, C₃₋₈ cycloalkyl group such as cyclopentyl and cyclohexyl, C₆₋₁₂ aryl group such as phenyl and α-naphthyl, C₇₋₁₄ aralkyl group such as phenyl-C₁₋₂ alkyl group such as benzyl and phenetyl or α-naphthyl-C₁₋₂ alkyl group such as α-naphthylmethyl, as well as pivaloyloxymethyl group generally used in an oral ester are used.

When the present receptor protein has carboxyl group (or carboxylate) at a position other than a C-terminal, proteins in which carboxyl group is amidated or esterified are also included in the present receptor protein. As an ester in this case, for examples, an ester at a C-terminal described above is used.

Further, the present receptor proteins include proteins in which an amino group of a methionine residue of a N-terminal amino acid residue (for example, methionine residue is protected with a protecting group (for example, C₁₋₆ acyl group such as C₁₋₆ alkanoyl such as formyl group and acetyl group) in the aforementioned proteins, proteins in which a N-terminal glutamine residue produced by cutting in the living body is pyroglutamine-oxidized, proteins in which a substituent (such as -OH, -SH, amino group, imidazole group, indole group, guanidino group) on a side chain of an intramolecular amino acid is protected with a suitable protecting group (C₁₋₆ acyl group such as C₁₋₆ alkanoyl group such as formyl group and acetyl group) , and conjugated proteins such as glycoprotein having sugar chains bound thereto.

As an embodiment of the present receptor protein, for example, a receptor protein comprising an amino acid sequence represented by SEQ ID No:1, a receptor protein comprising an amino acid sequence represented by SEQ ID No:2, and a receptor protein comprising an amino acid sequence represented by SEQ ID No:7 are used.

As a partial peptide of the present receptor protein (hereinafter abbreviated as partial peptide in some cases) , any partial peptides are used as long as they are a partial peptide of the aforementioned receptor protein. For example, among the present receptor protein molecules, a partial peptide which is exposed outside a cell membrane and has the receptor binding activity is used.

More specifically, as a partial peptide of a receptor protein having an amino acid sequence represented by SEQ ID No:1, there is a peptide comprising a part analyzed to an extracellular region (hydrophilic site) in a hydrophobic plot analysis shown in FIG. 1. In addition, a peptide comprising a hydrophobic site as its part may be also used. Although peptides comprising individual domains separately may be used, peptides of a part comprising simultaneously a plurality of domains may be used.

It is preferable that the number of amino acids of the present partial peptide is at least 20 or more, preferably 50 or more, more preferably 100 or more among component amino acid sequence of the aforementioned present receptor protein.

Substantially same amino acid sequence denotes an amino acid sequence having about 50% or more, preferably about 70% or more, more preferably about 80% or more, further preferably about 90% or more, most preferably about 95% or more homology with these amino acid sequences.

Here, the terms "substantially homogeneous properties" have the same meanings as defined above. Measurement of "substantially homogeneous properties" can be performed as described above.

In addition, in the present partial peptide, 1 or 2 or more (preferably, around 1 to 10, more preferably a few (1 to 5)) amino acids in the aforementioned amino acid sequence may be deleted, or 1 or 2 or more (preferably, around 1 to 20, more preferably around 1 to 10, further preferably a few (1 to 5)) amino acids may be added to the amino acid sequence, or 1 or 2 or more (preferably, around 1 to 20, more preferably 1 to 10, further preferably a few (1 to 5)) amino acids may be substituted with other amino acids.

In addition, the present partial peptide has usually a carboxyl group (-COOH) or carboxylate (-COO⁻) as a C-terminal. However, a C-terminal may be amido (-CONH₂) or an ester (-COOR) as in the present protein.

Further, partial peptides in which an amino group of a methionine group of a N-terminal is protected with a protecting group, partial peptides in which a N-terminal is cut in the living body and the produced Gln is pyroglutamine-oxidized, partial peptides in which a substituent on a side chain of an intramolecular amino acid is protected with a suitable protecting group, or a conjugated peptide in which a sugar chain is bound, such as so-called glycopeptide are included in the present peptide, as in the aforementioned present receptor protein.

In addition, although the present partial peptide has usually a carboxyl group (-COOH) or carboxylate (-COO⁻) as a C-terminal, a C-terminal may be an amido (-CONH₂) or an ester (-COOR) as in the aforementioned present protein.

Examples of salts of the receptor protein or partial peptide of the present invention include salts with physiologically acceptable acids (e.g., inorganic acids, organic acids) or bases (e.g., alkali metal salts), and preferably physiologically acceptable acid addition salts. Such salts include, for example, salts with inorganic acids (e.g., hydrochloric acid, phosphoric acid, hydrobromic acid, sulfuric acid) or salts with organic acids (e.g., acetic acid, formic acid, propionic acid, fumaric acid, maleic acid, succinic acid, tartaric acid, citric acid, malic acid, oxalic acid, benzoic acid, methanesulfonic acid, benzenesulfonic acid).

The present receptor protein or a salt thereof can be prepared from cells or tissues of a human being and a warm blood mammal described above by the method of purifying proteins known per se, or can be prepared by culturing a transformant comprising a DNA encoding a protein described later. Alternatively, it can be prepared according to a method of synthesizing a peptide described later.

For production from human or mammalian tissues or cells, the human or mammalian tissues or cells are homogenized and then extracted with e.g. an acid, and the extract can be subjected to purification and isolation by a combination of chromatographic techniques such as reverse-phase chromatography, ion-exchange chromatography etc.

For synthesis of the receptor protein or partial peptide of the present invention or a salt thereof or amide derivatives thereof, usually commercially available resin for protein synthesis can be used. Such resin includes, for example, chloromethyl resin, hydroxymethyl resin, benzhydryl amine resin, aminomethyl resin, 4-benzyloxybenzyl alcohol resin, 4-methylbenzhydryl amine resin, PAM resin, 4-hydroxymethylmethylphenylacetamidemethyl resin, polyacrylamide resin, 4-(2',4'-dimethoxyphenyl-hydroxymethyl) phenoxy resin, 4-(2',4'-dimethoxyphenyl-Fmoc aminoethyl) phenoxy resin, and so forth. On the resin described above, each amino acid with the α-amino group and side-chain functional group properly protected is condensed sequentially in accordance with the sequence of the desired protein by the per se known condensation methods. At the end of the reaction, the protein is cleaved off from the resin while various protecting groups are removed, and the product is subjected to a reaction of forming intramolecular disulfide bonds in a highly dilute solution to give the desired protein or an amide thereof.

A wide variety of activating reagents usable for protein synthesis can be used for condensation of the protected amino acids described above, and carbodiimides are particularly preferable. Examples of such carbodiimides include DCC, N,N'-diisopropylcarbodiimide, N-ethyl-N'-(3-dimethylaminoprolyl)carbodiimide, etc. For activation by these reagents, the protected amino acids along with racemization inhibitors (e.g., HOBt, HOOBt) can be added to the resin directly or after the protected amino acids were previously activated as symmetric acid anhydrides or HOBt esters or HOOBt esters.

The solvent used for activation of each protected amino acid or for condensation thereof with the resin can be selected as necessary from those solvents known to be usable in protein condensation reaction. Examples of such solvent include acid amides such as N,N-dimethylformamide, N,N-dimethylacetamide and N-methylpyrrolidone, halogenated hydrocarbons such as methylene chloride and chloroform, alcohols such as trifluoroethanol, sulfoxides such as dimethyl sulfoxide, ethers such as pyridine, dioxane and tetrahydrofuran, nitriles such as acetonitrile and propionitrile, esters such as methyl acetate and ethyl acetate, or a suitable mixture thereof. The reaction temperature is usually selected as necessary within the range known to be usable in the reaction of forming protein bonds, and usually the reaction temperature is selected within the range of about -20 °C to 50 °C. The activated amino acid derivatives are used usually in excess (1.5- to 4-fold). When their condensation is insufficient, their sufficient condensation is achieved as a result of a test using ninhydrin reaction by repeatedly carrying out the condensation reaction without conducting elimination of the protecting groups. When their sufficient condensation is not achieved even by repeatedly carrying out the reaction, the unreacted amino acids are acetylated with acetic anhydride or acetyl imidazole.

The protecting groups for amino groups in the starting materials include, for example, Z, Boc, t-pentyloxycarbonyl, isobornyloxycarbonyl, 4-methoxybenzyloxycarbonyl, Cl-Z, Br-Z, adamantyloxycarbonyl, trifluoroacetyl, phthaloyl, formyl, 2-nitrophenylsulphenyl, diphenylphosfinothioyl, Fmoc etc.

The carboxyl group can be protected by, for example, alkyl esterification (e.g., straight-chain, branched or cyclic alkyl esterification such as methyl, ethyl, propyl, butyl, t-butyl, cyclopentyl, cyclohexyl, cycloheptyl, cycloctyl or 2-adamantyl esterification), aralkyl esterification (e.g., benzyl esterification, 4-nitrobenzyl esterification, 4-methoxybenzyl esterification, 4-chlorobenzyl esterification, benzhydryl esterification), phenacyl esterification, benzyloxycarbonylhydrazidation, t-butoxycarbonylhydrazidation, tritylhydrazidation etc.

The hydroxyl group in serine can be protected by, for example, esterification or etherification. A suitable group used in this esterification includes, for example, lower alkanoyl groups such as acetyl group, alloyl groups such as benzoyl group, and carbonic acid-derived groups such as benzyloxycarbonyl group and ethoxycarbonyl group. A suitable group for etherification includes, for example, a benzyl group, tetrahydropyranyl group, t-butyl group etc.

The protecting group used for the phenolic hydroxyl group in tyrosine includes, for example, Bzl, Cl₂-Bzl, 2-nitrobenzyl, Br-Z, t-butyl etc.

The protecting group used for imidazole in histidine includes, for example, Tos, 4-methoxy-2,3,6-trimethylbenzenesulfonyl, DNP, benzyloxymethyl, Bum, Boc, Trt, Fmoc etc.

Activated carboxyl groups in the starting materials include, for example, the corresponding acid anhydrides, azides and active esters (i.e. esters with alcohols such as pentachlorophenol, 2,4,5-trichlorophenol, 2,4-dinitrophenol, cyanomethylalcohol,p-nitrophenol,HONB,N-hydroxysuccinamide, N-hydroxyphthalimide and HOBt). The activated amino groups in the starting materials include, for example, the corresponding phosphoric acid amides.

Examples of methods for removing'(leaving) of the protecting groups include catalytic reduction in a hydrogen stream in the presence of a catalyst such as Pd-black or Pd-carbon, acid treatment using anhydrous hydrogen fluoride, methane sulfonic acid, trifluoromethane sulfonic acid, trifluoroacetic acid or a mixed solution thereof, base treatment using diisopropylethylamine, triethylamine, piperidineorpiperazine, and reduction using sodium in liquid ammonia. The leaving reaction by the acid treatment described above is carried out generally at a temperature of about -20 °C to 40 °C, and it is effective in the acid treatment to add a cation scavenger such as anisole, phenol, thioanisole, m-cresol, p-cresol, dimethylsulfide, 1,4-butanedithiol and 1,2-ethanedithiol. A 2,4-dinitrophenyl group used as a protecting group for imidazole in histidine can also be removed by treatment with thiophenol, while a formyl group used as a protecting group for indole in tryptophan can be removed not only by deprotection by acid treatment in the presence of 1,2-ethanedithiol or 1,4-butanedithiol, but also by alkali treatment using dilute sodium hydroxide solution or dilute ammonia.

Protection and protecting groups for functional groups which should not participate in the reaction of the starting materials, elimination of the protecting groups, and activation of functional groups participating in the reaction can be selected as necessary from known groups or known means.

Another method of obtaining an amide derivative of the protein includes, for example, protecting the α-carboxyl group of a C-terminal amino acid by amidation, then extending a peptide (protein) chain at the side of the amino group until it attains desired chain length, and thereafter producing a protein of said peptide chain from which only the protecting group for the N-terminal α-amino group was removed and a protein of said peptide chain from which only the protecting group for the C-terminal carboxyl group was removed, followed by condensation both the proteins in the mixed solvent as described above. The details of the condensation reaction are the same as described above. The protected protein obtained by condensation is purified, and every protecting group is removed by the method descried above, whereby the desired crude protein can be obtained. This crude protein is purified by a wide variety of known purification techniques, and by lyophilizing its major fraction, the desired amide derivative of the protein can be obtained.

To obtain an ester derivative of the protein, for example the α-carboxyl group of a C-terminal amino acid is condensed with desired alcohol to form an amino acid ester from which the desired ester derivative of the protein can be obtained in the same manner as for the amide derivative of the protein.

The partial peptide of the present invention or a salt thereof can be produced according to a peptide synthesis per se known method or by cleaving the protein of the present invention with a suitable peptidase. For example, the peptide synthesis method may be the solid- or liquid-phase synthesis method. That is, the desired peptide can be obtained by condensation of a partial peptide or amino acids capable of constituting the partial peptide of the present invention with the remainder, followed by elimination of protecting groups if any from the product. As the known condensation method and the elimination of the protecting groups, mention is made of e.g. the methods described in (1) to (5) below:
(1) M. Bodanszky and M. A. Ondetti, Peptide Synthesis, Interscience Publisher, New York (1966);
(2) Schroeder and Luebke, The Peptide, Academic Press, New York (1965);
(3) Nobuo Izumiya et al., Basis and Experiments in Peptide Synthesis (in Japanese), Maruzen Co., Ltd. (1975);
(4) Haruaki Yajima and Shunpei Sakakibara, Biochemical Experimental Course 1, Protein Chemistry IV, 205, (1977); and
(5) Haruaki Yajima (supervisor), Development of medicines, a second series, vol.14, Peptide Synthesis, Hirokawashoten.

After the reaction, the partial peptide of the present invention can be isolated and purified by a combination of conventional purification techniques such as solvent extraction, distillation, column chromatography, liquid chromatography and recrystallization. If the partial peptide is obtained in a free form by these methods, the product can be converted into a suitable salt by a known method or its analogous method, or if the partial peptide is obtained in a salt form, it can be converted into a free peptide or other salts by a known method or its analogous method.

As a polynucleotide encoding the present receptor protein, any polynucleotides (DNA or RNA, preferably DNA) may be used as long as they comprise a base sequence encoding the aforementioned presentreceptorprotein. As the polynucleotide, there are a DNA and a RNA such as a mRNA encoding the present receptor protein, whether double-stranded or single-stranded. In the case of the double-strand, a double-stranded DNA, a double-stranded RNA or a DNA:RNA hybrid may be used. In the case of single-strand, a sense strand (that is, coding strand), or an antisense strand (that is, non-coding strand) may be used.

A mRNA for the present receptor protein can be quantitated using a polynucleotide encoding the present receptor protein, for example, by a method described in the known Experimental Medicine, Extra Number "New PCR and its Application" 15(7), 1997 or the similar method.

As a DNA encoding the present receptor protein, any of a genomic DNA, a genomic DNA library, a cDNA derived from the aforementioned cells or tissues, a cDNA library derived from the aforementioned cells or tissues, and a synthetic DNA may be used. A vector used for a library may be any of bacteriophage, plasmid, cosmid and phagemide. In addition, amplification can be performed directly by Reverse Transcriptase Polymerase Chain Reaction (hereinafter abbreviated as RT-PCT method) using total RNA or mRNA fraction prepared from the aforementioned cells or tissues.

Specifically, as a DNA encoding the present receptor protein, for example, a DNA comprising a base sequence represented by SEQ ID No: 3, SEQ ID NO:4 or SEQ ID No:8, or any DNAs having a base sequence which hybridizes with a base sequence represented by SEQ ID No: , SEQ ID NO:4 or SEQ ID No:8 under the highly stringent conditions, and having the substantially homogeneous properties (for example, ligand binding activity and signal information transmission action) as that of the present receptor protein may be used.

As a DNA which can hybridize with a base sequence represented by SEQ ID No:3, SEQ ID No:4 or SEQ ID No:8, for example, a DNA comprising a base sequence having about 70% or more, preferably about 80% or more, more preferably about 90% or more, most preferably about 95% or more homology with a base sequence represented by SEQ ID No:3, SEQ ID No:4 or SEQ ID No:8 is used.

Hybridization can be carried out according to a per se known method or its analogous method, for example a method described in Molecular Cloning, 2nd ed. (J. Sambrook et al., Cold Spring Harbor Lab. Press, 1989). If a commercial library is used, hybridization can be carried out according to the manufacture's instructions. Preferably, hybridization can be conducted under high stringent conditions.

The high stringent conditions refer to those conditions under which the concentration of sodium is about 19 to 40 mM, preferably about 19 to 20 mM, and the temperature is about 50 to 70 °C, preferably about 60 to 65 °C.

More specifically, as a DNA encoding a receptor protein comprising an amino acid sequence represented by SEQ ID No:1, a DNA comprising a base sequence represented by SEQ ID No:3 is used. As a DNA encoding a receptor protein comprising an amino acid sequence represented by SEQ ID No:2, a DNA comprising a base sequence represented by SEQ ID No:4 is used. As a DNA encoding a receptor protein comprising an amino acid sequence represented by SEQ ID No:7, a DNA comprising a base sequence represented by SEQ ID No:8 is used.

A polynucleotide comprising a part of a base sequence of a DNA encoding the present receptor protein, or a part of a base sequence complementary with the DNA means not only include a DNA encoding the following present partial peptide but also include RNA.

According to the present invention, an antisense polynucleotide (nucleic acid) which can inhibit replication or expression of a G protein coupled receptor protein gene can be designed and synthesized based on a base sequence information of a DNA encoding the cloned or determined G protein coupled receptor protein. Such the polynucleotide (nucleic acid) can hybridize with a RNA for a G protein coupled receptor protein gene, can inhibit the synthesis or the functions of the RNA, or can regulate or control expression of a G protein coupled receptor protein gene via interaction with a G protein coupled receptor protein-related RNA. A polynucleotide complementary with a selected sequence of a G protein coupled receptor protein-related RNA, and a polynucleotide which can specifically hybridize with a G protein coupled receptor protein-related RNA are useful for regulating and controlling expression of a G protein coupled receptor protein gene in vivo and in vitro, and useful for treating or diagnosing diseases. A term "correspond" means homologous or complementary with a particular sequence of a nucleotide, a base sequence or nucleic acid including a gene. "Correspond" between a nucleotide, a base sequence or a nucleic acid and a peptide (protein) usually denotes an amino acid of a peptide (protein) under direction induced by a nucleotide (nucleic acid) sequence or its complement. Although 5'-terminal hairpin roop, 5'-terminal 6-basepair repeat, 5'-terminal nontranslation region, polypeptide translation initiating codon, protein coding region, ORF translation initiating codon, 3'-terminal nontranslation region, 3'-terminal palindrome region and 3'-terminal hairpin roop can be selected as a preferable subject region, any region in a G protein coupled receptor gene as a subject region.

The relationship between an end nucleic acid and polynucleotide complementary with at least a part of a subject region, and the relationship between a subject and a polynucleotide which can hybridize therewith can be said to be "antisense". As an antisense polynucleotide, there are a polydeoxynucleotide comprising 2-deoxy-D-ribose, a polydeoxynucleotide comprising D-ribose, other type polynucleotide which is a N-glycoside of a purine or pyrimidine base, or other polymer having a non-nucleotide skeleton (for example, a commercially available protein nucleic acid and synthetic sequence specific nucleic acid polymer) or other polymer comprising a special linkage (providied that, the polymer contains their nucleotide having arrangement permitting base pairing and base attachment found in a DNA and a RNA). They may be a double-stranded DNA, a single-stranded DNA, a double-stranded RNA, a single-stranded RNA, or a DNA: RNA hybrid, a non-modified polynucleotide (or non-modified oligonucleotide) or may have the added known modification, for example, may have a label known in the art, or have a cap, or may be methylated, or may have substitution of 1 or more natural nucleotide with similar nucleotides, or may have intramolecular nucleotide modification, for example, may have non-charged linkage (methylphosphonate, phosphotriester, phosphoramidate, and carbanate), may have a charged linkage or a sulfur-comprising linkage (for example, phosphorothioate and phosphorodithioate), for example, may have a side chain group of a protein (nuclease, a nuclease inhibitor, toxin, antibody, signal peptide, poly-L-lysine) or a sugar (for example, monosaccharide), may have intercalent compound (for example, acridine and psoralen), may contain a chelate compound (for example, metal, radioactive metal, boron, oxidative metal), may contain alkylating agent, or may have a modified linkage, (for example, α anomer type nucleic acid). Here, "nucleoside", "nucleotide" and "nucleic acid" include not only those comprising a purine and pyrimidine base but also those having modified other heterocyclic type base. Such the modified materials may contain methylated purine and pyrimidine, acylated purine and pyrimidine, or other heterocycle. A modified nucleotide and a modified nucleotide may have a modified sugar part and, for example, 1 or more hydroxy groups may be substituted with halogen or an aliphatic group, or converted into a functional group such as ether and amine.

The present antisense polynucleotide (nucleic acid) is a RNA, a DNA, or a modified nucleic acid (RNA, DNA). Examples of the modified nucleic acid are not limited to but include a sulfur derivative and a thiophosphate derivative of nucleic acid, and modified nucleic acids which are resistant to degradation of polynucleosideamide and oligonucleosideamide. The present antisense nucleic acid can be preferably designed under the following strategy. That is, an antisense nucleic acid in a cell is made to be more stable, the cell permeability of an antisense nucleic acid is enhanced, affinity for a goal sense strand is made to be greater and, if any, toxicity of an antisense nucleic acid is made to be smaller.

A variety of such the modifications are known in the art. For example, there is a disclosure in G. Kawakami et al., Pharm. Tech. Japan, vol. 8, pp. 247, 1992; vol. 8, pp.395, 1992; S. T. Crooke et al. ed., Antisense Research and Applications, CRS Press, 1993.

The present antisense nucleic acid may be altered, may contain a modified sugar, a base or a linkage, may be supplied in the special form of a liposome and a microsphere, may be applied by gene theraphy, may be given in the addition form. Examples of the antisense nucleic acid which may be used in the addition form include a polycation such as polylysine which neutralizes a charge of phosphate skeleton, and hydrophobic material such as a lipid which enhances the interaction with the cell membrane and increases uptake of nucleic acid (for example, phospholipid and cholesterol) . Examples of a preferable lipid to be added include cholesterol and its derivative (for example, cholesteryl chloroformate and cholic acid). These can be attached to a 3'-terminal or a 5'-terminal of nucleic acid and can be attached via a base, a sugar, and an intramolecular nucleoside linkage. An example of other group includes a group for capping and preventing degradation by a nuclease such as exonuclease and RNase. An example of such the group for capping is not limited to but includes a glycol such as a protecting group for a hydroxyl group known in the art including polyethylene glycol, tetraethylene glycol.

The inhibitory activity of an antisense nucleic acid can be examined by using the present transformant, the present in vivo and in vitro gene expression system, or in vivo and in vitro translation system for a G protein coupled receptor protein. The nucleic acid can be applied to a cell by a variety of per se known methods.

As a DNA encoding the present partial peptide, any DNAs may be used as long as they contain a base sequence encoding the aforementioned present partial peptides and, in addition, the DNA may be a genomic DNA, a genomic DNA library, a cDNA derived from the aforementioned cells or tissues, a cDNA library derived from the aforementioned cell or tissue, or a synthetic DNA. A vector used in a library may be any of bacteriophase, plasmid, cosmid, and phagemid. In addition, the DNA may be amplified directly by Reverse Transcriptase Polymerase Chain Reaction (hereinafter abbreviated as RT-PCR method) using a mRNA fraction prepared from the aforementioned cells and tissues.

More particularly, as a DNA encoding the present partial peptide, for example, (1) a DNA having a partial base sequence of a DNA having a base sequence represented by SEQ ID No:3, SEQ ID No:4 or SEQ ID No:8, or (2) a DNA having a base sequence which hybridizes with a base sequence represented by SEQ ID No: , SEQ ID No:4 or SEQ ID No:8 under the highly stringent conditions, and having a partial base sequence of a DNA encoding a receptor protein having the substantially homogeneous properties (for example, ligand binding activity and signal information transmission action) to that of the present receptor protein peptide are used.

A DNA which can hybridize with a base sequence represented by SEQ ID No:3, SEQ ID No:4, or SEQ ID No:8, for example, a DNA comprising a base sequence having about 70% or more, preferably about 80% or more, more preferably about 90% or more, most preferably about 95% or more homology with a base sequence represented by SEQ ID No:3, SEQ ID No:4, or SEQ ID No:8 is used.

As a means of cloning the DNA encoding completely for the receptor protein and partial peptide of the present (hereinafter abbreviated as present receptor protein in some cases), it is possible to use amplification by the PCR method using synthetic DNA primers having a partial base sequence of the present receptor protein as well as selection by hybridization of the DNA integrated in a suitable vector with a labeled DNA fragment or synthetic DNA encoding for a part or the whole of the present receptor protein. Hybridization can be carried out according to a method described in, for example, Molecular Cloning, 2nd ed. (J. Sambrook et al., Cold Spring Harbor Lab. Press, 1989). If a commercial library is used, hybridization can be carried out according to the manufacture's instructions.

Conversion of DNA into a base sequence can be carried out by a per se known method, for example by the Gupped duplex method or Kunkel method or its analogous method by using PCR or a known kit such as Mutant**™**-G (Takara Shuzo Co., Ltd.) or Mutant™-K (Takara Shuzo Co., Ltd.).

A DNA encoding a cloned receptor protein can be used as it is or, if desired, by digesting with a restriction enzyme or adding a linker thereto. The DNA may have ATG as a translation initiation codon at the 5'-terminal thereof and TAA, TGA or TAG as a translation termination codon at the 3'-terminal thereof. These translation initiation and termination codons can also be added to the DNA via a suitable synthetic DNA adaptor.

An expression vector for the present receptor can be produced for example by (A) cutting the desired DNA fragment off from the DNA encoding for the protein of the present invention and then (B) ligating said DNA fragment to a region downstream from a promoter in a suitable expression vector.

The vector used includes E. coli-derived plasmid (e.g., pBR322, pBR325, pUC12, pUC13), Bacillus subtilis-derived plasmid (e.g., pUB110, pTP5, pC194), yeast-derivedplasmid (e.g., pSH19, pSH15), bacteriophage such as λ-phage, and animal viruses such as retrovirus, vaccinia virus and Baculovirus, as well as pA1-11, pXT1, pRc/CMV, pRc/RSV, pcDNAI/Neo etc.

The promoter used in the present invention may be any suitable promoter compatible with a host used for expression of the gene. For example, when animal cells are used as the host, mention is made of SRα promoter, SV40 promoter, HIV-LTR promoter, CMV promoter, HSV-TK promoter etc.

Among these promoters, CMV (cytomegalovirus) promoter, SRα promoter etc. are preferably used. It is preferable to use trp promoter, lac promoter, recA promoter, λPL promoter, lpp promoter, T7 promoter etc. for microorganisms of the genus Escherichia as the host, SPO1 promoter, SPO2 promoter, penP promoter etc. for microorganisms of the genus Bacillus as the host, and PHO5 promoter, PGKpromoter, GAP promoter, ADHpromoter etc. for yeasts as the host. When insect cells are used as the host, polyhedron promoter, P10 promoter etc. are preferable.

The expression vector may contain an enhancer, a splicing signal, a poly A-added signal, a selective marker, an SV40 origin of replication (also referred to hereinafter as SV40 ori) etc. in addition to the promoter described above. The selective marker includes, for example, dihydrofolate reductase (also referred to hereinafter as dhfr) gene [methotrexate (MTX) resistance], ampicillin resistance gene (also referred to hereinafter as Amp^{r}) and neomycin resistance gene (G418 resistance, also referred to hereinafter as Neo^{r}). In particular, if the dhfr gene is used as a selective marker for dhfr gene-defective Chinese hamster cells, the desired gene can also be selected in a thymidine-free medium.

A signal sequence compatible with the host is added as necessary to a base sequence for the N-terminal of the present receptor protein. A PhoA signal sequence, Omp A signal sequence etc. can be utilized for microorganisms of the genus Escherichia used as the host; an α-amylase signal sequence, subtilisin signal sequence etc. for microorganisms of the genus Bacillus as the host; an MFα signal sequence, SUC2 signal sequence etc. for yeasts as the host; and an insulin signal sequence, α-interferon signal sequence, antibody molecule signal sequence etc. for animal cells as the host.

The thus constructed vector containing the DNA encoding for the present receptor protein can be used to produce transformants.

The microorganisms of the genus Escherichia, the microorganisms of the genus Bacillus, yeasts, insect cells, insects, animal cells etc. are used as the host.

The microorganisms of the genus Escherichia used include, for example, Escherichia coli K12 DH1 (Proc. Natl. Acad. Sci. USA, 60, 160 (1968)), JM103 (Nucleic Acids Research, 9, 309 (1981)), JA221 (Journal of Molecular Biology, 120, 517 (1978)), HB101 (Journal of Molecular Biology, 41, 459 (1969)), C600 (Genetics, 39, 440 (1954)), etc.

The microorganisms of the genus Bacillus used include, for example, Bacillus subtilis MI114 (Gene, 24, 255 (1983)), 207-21 (Journal of Biochemistry, 95, 87 (1984)), etc.

The yeasts used include Saccharomyces cerevisiae AH22, AH22R⁻, NA87-11A, DKD-5D, 20B-12, Schizosaccharomyces pombe NCYC1913, NCYC2036, Pichia pastoris KM71, etc.

The insect cells used when the virus is AcNPV include, for example, cells (Spodoptera frugiperda cells; Sf cells) from an established cell line derived from caterpillars of Spodoptera frugiperda, MG1 cells derived from the midgut in Trichoplusia ni, High Five™ cells derived from eggs of Trichoplusia ni, cells derived from Mamestra brassicae and cells derived from Estigmena acrea. When the virus is BmNPV, cells (Bombyx mori N cells; BmN cells) from an established cell line derived from silkworms, etc., are used. The Sf cells used include, for example, Sf9 cells (ATCC CRL1711), Sf21 cells (Vaughn, J. L. et al., In Vivo, 13, 213-217 (1977)), etc.

The insects used include, for example, silkworm caterpillars (Maeda et al., Nature, 315, 592 (1985)).

The animals cells used include, for example, simian cell COS-7, Vero, Chinese hamster ovary cells (abbreviated hereinafter to CHO cells), dhfr gene-defect Chinese hamster ovary cells (abbreviated hereinafter to CHO (dhfr⁻) cells), mouse L cells, mouse AtT-20, mouse myeloma cells, rat GH3, human FL cells etc.

The microorganisms of the genus Escherichia can be transformed according to a method described in e.g. Proc. Natl. Acad. Sci. USA, 69, 2110 (1972) or Gene, 17, 107 (1982). The microorganisms of the genus Bacillus can be transformed according to a method described in e.g. Molecular & General Genetics, 168, 111 (1979), etc.

The yeasts can be transformed according to a method described in e.g. Methods in Enzymology, 194, 182-187 (1991), Proc. Natl. Acad. Sci. USA, 75, 1929 (1978), etc.

The insect cells or insects can be transformed according to a method described in e.g. Bio/Technology, 6, 47-55 (1988), etc.

The animal cells can be transformed according to a method described in e.g. "Saibo Kogaku Bessatsu 8, Shin-Saibo Kogaku Jikken Protocol (Cell Technology, Extra Number 8, New Experimental Protocol in Cell Technology)", 263-267 (1995) (published by Shujunsha), Virology, 52, 456 (1973), etc.

Thereby, a transformant transformed with an expression vector comprising a DNA encoding a G protein coupled receptor protein can be obtained.

When transformants derived from the microorganisms of the genus Escherichia or Bacillus as the host are cultured, the medium used for their culture is preferably a liquid medium containing a carbon source, a nitrogen source, inorganic matter etc. necessary for growth of the transformants. The carbon source includes, for example, glucose, dextrin, soluble starch, sucrose etc.; the nitrogen source includes, for example, inorganic or organic materials such as ammonium salts, nitrates, corn steep liquor, peptone, casein, meat extract, soybean cake and potato extract; and the inorganic matter includes, for example, calcium chloride, sodium dihydrogen phosphate, magnesium chloride etc. In addition, yeast, vitamins, growth promoters etc. may be added. The pH value of the medium is desirably about 5 to 8.

For example, the medium for culturing the microorganisms of the genus Escherichia is preferably M9 medium containing glucose and casamino acid (Miller, Journal of Experiments in Molecular Genetics, 431-433, Cold Spring Harbor Laboratory, New York, 1972). To permit the promoter to work efficiently, a chemical such as 3β-indolylacrylic acid can be added as necessary.

The transformants from the microorganisms of the genus Escherichia as the host are cultured usually at about 15 to 43° C for about 3 to 24 hours during which the medium may be aerated or stirred as necessary.

The transformants from the microorganisms of the genus Bacillus as the host are cultured usually at about 30 to 40° C for about 6 to 24 hours during which the medium may be aerated or stirred as necessary.

The medium used for culturing the transformants from yeasts as the host includes, for example, Burkholder minimum medium (Bostian, K. L. et al., Proc. Natl. Acad. Sci. USA, 77, 4505 (1980)) and SD medium containing 0.5% casamino acid (Bitter, G. A. et al., Proc. Natl. Acad. Sci. USA, 81, 5330 (1984)). The pH value of the medium is adjusted preferably to about 5 to 8. The transformants are cultured usually at about 20 to 35°C for about 24 to 72 hours during which the medium may be aerated or stirred as necessary.

The medium used for culturing the transformants from insect cells or insects as the host includes, for example, a medium, if necessary prepared by adding inactivated additives such as 10% bovine serum to Grace's insect medium (Grace, T. C. C., Nature, 195, 788 (1962)). The pH value of the medium is adjusted preferably to about 6.2 to 6.4. The transformants are cultured usually at about 27°C for about 3 to 5 days during which the medium may be aerated or stirred as necessary.

The medium used for culturing the transformants from animal cells as the host includes, for example, MEM medium containing about 5 to 20% fetal bovine serum (Science, 122, 501 (1952)), DMEM medium (Virology, 8, 396 (1959)), RPMI 1640 medium (The Journal of the American Medical Association, 199, 519 (1967)), 199 medium (Proceeding of the Society for the Biological Medicine, 73, 1 (1950)) etc. The pH value is preferably about 6 to 8. The transformants are cultured usually at about 30 to 40 °C for about 15 to 60 hours during which the medium may be aerated or stirred as necessary.

As described above, the present G protein coupled receptor protein can be formed on cytoplasmic membranes of the transformants.

In order to separate the present receptor protein from the above culture and purify the protein, this can be performed, for example, by the following method.

To extract the present receptor protein from the cultured microorganisms or cells, the cultured microorganisms or cells are collected in a usual manner, suspended in a suitable buffer, disrupted by sonication, lysozyme and/or freezing and thawing, and centrifuged or filtered to give a crude extract of the receptor protein. The buffer may contain receptor protein denaturants such as urea and guanidine hydrochloride and surfactants such as Triton X-100™. When the receptor protein is secreted into the culture liquid, the culture supernatant is collected by separating the supernatant from the cultured microorganisms or cells by a per se known method.

The culture supernatant thus obtained, or the receptor protein contained in the extract, can be purified by a suitable combination of separation and purification techniques known per se. These known separation and purification techniques make use of a method of utilizing solubility, such as salting-out and solvent precipitation, a method of mainly utilizing a difference in molecular weight, such as dialysis, ultrafiltration, gel filtration, and SDS-polyacrylamide gel electrophoresis, a method of utilizing a difference in electric charge, such as ion-exchange chromatography, a method of utilizing specific affinity, such as affinity chromatography, a method of utilizing a difference in hydrophobicity, such as reverse-phase HPLC, a method of utilizing a difference in isoelectric point, such as isoelectric focusing.

If the receptor protein thus obtained is in a free form, it can be converted into a salt by a per se known method or its analogous method, while if the resulting receptor protein is obtained in the form of a salt, it can be converted into a free receptor protein or another salt by a per se known method or its analogous method.

Before or after purification, the receptor protein produced by the transformants can be arbitrarily modified or its partial polypeptide can be removed by allowing a suitable protein-modifying enzyme to act on the protein. For example, trypsin, chymotrypsin, arginyl endopeptidase, protein kinase or glycosidase is used as the protein-modifying enzyme.

The thus formed present receptor protein or a salt thereof can be measured by enzyme immunoassays or Western blotting with specific antibody.

The antibody against the present receptor protein and partial peptide or a salt thereof may be a polyclonal or monoclonal antibody capable of recognizing the present receptor protein or partial peptide or a salt thereof.

The antibody against the present receptor protein and partial peptide or a salt thereof (hereinafter abbreviated as present receptor protein and the like in some cases) can be produced by a known process for producing antibody or antiserum by using the present receptor protein as the antigen.

### [Preparation of a monoclonal antibody]

### (a) Preparation of monoclonal antibody-producing cells

The present receptor protein is administered alone or together with a carrier or a diluent into mammals at a site where the antibody can be produced by administration. To enhance the ability of the animals upon administration to produce the antibody, complete Freund's adjuvant or incomplete Freund's adjuvant may be administered. Administration is conducted usually once every 2 to 6 weeks and about 2 to 10 times in total. The mammals used include, for example, monkey, rabbit, dog, guinea pig, mouse, rat, sheep and goat. Among others, mouse and rat are preferably used.

For production of the monoclonal antibody-producing cells, those animals having antibody titer are selected from the warm-blooded animals (e.g. mice) immunized with the antigen, and on the second to fifth day after the final immunization, their spleens or lymph nodes are collected, and the antibody-producing cells contained therein are fused with myeloma cells from animals of the same or different species, whereby monoclonal antibody-producing hybridoma can be produced. The antibody titer in antiserum can be measured for example by reacting the antiserum with the labeled protein described below and then measuring the activity of the labeling agent bound to the antibody. Fusion can be carried out by a known method such as the method of Keller and Millstein (Nature, 256, 495 (1975)). The fusion promoter includes polyethylene glycol (PEG) andSendai virus, and PEG is preferably used.

The myeloma cells include myeloma cells from warm-blooded animals, such as NS-1, P3U1 and SP2/0 among which P3U1 is preferably used. The ratio of the antibody-producing cells (spleen cells) to the myeloma cells used is from 1 : 1 to 20 : 1, and cell fusion can be effected efficiently by incubating the cells for about 1 to 10 minutes at 20 to 40 °C, preferably 30 to 37 °C, in the presence of PEG (preferably PEG 1000 to PEG 6000) at a concentration of about 10 to 80%.

The monoclonal antibody-producing hybridoma can be screened by various methods, for example by adding a culture supernatant of the hybridoma to a solid phase (e.g., a microplate) having the antibody of the receptor protein, etc adsorbed thereon directly or along with a carrier and then adding a radioactive substance- or enzyme-labeled anti-immunoglobulin antibody (which is e.g. an anti-mouse immunoglobulin antibody when mouse cells are subjected to cell fusion) or protein A to detect the monoclonal antibody bound to the solid phase or by adding a culture supernatant of the hybridoma to a solid phase having an anti-immunoglobulin antibody or protein A adsorbed thereon and then adding the receptor protein labeled with a radioactive substance or an enzyme to detect the monoclonal antibody bound to the solid phase.

The monoclonal antibody can be screened according to a per se known method or its analogous manner. Screening can be carried out usually in an animal cell culture medium to which HAT (hypoxanthine, aminopterin, thymidine) was added. The screening and breeding medium may be any medium in which the hybridoma can grow. Examples of such medium include PRMI 1640 medium containing 1 to 20% (preferably 10 to 20%) FBS, GIT medium containing 1 to 10% FBS (Wako Pure Chemical Industries, Ltd.), and a serum-free medium for hybridoma culture (SFM-101, Nissui Pharmaceutical, Co., Ltd.). The culture temperature is usually 20 to 40 °C, preferably about 37 °C. The culture time is usually 5 days to 3 weeks, preferably 1 to 2 weeks. Culture can be conducted usually in 5% CO₂ gas. The antibody titer in a culture supernatant of the hybridoma can be measured in the same manner as in the measurement of the antibody titer in antiserum as described above.

### (b) Purification of the monoclonal antibody

Separation and purification of a monoclonal antibody can be performed according to the per se known method, for example, a method of separating and purifying an immunoglobulin [such as a salting-out method, an alcohol precipitation method, an isoelectric precipitation method, an electrophoresis method, an adsorbing-desorbing method with an ion-exchanger (such as DEAE), an ultracentrifugation method, a gel filtration method, a specific purifying method of obtaining an antibody by taking only an antibody with an antigen binding solid phase or an active absorbing agent such as protein A or protein G, and dissociating a binding, as in the normal separation and purification of a polyclonal antibody.

### [Preparation of a polyclonal antibody]

A polyclonal antibody of the present invention can be prepared by the per se known method or its analogous method. For example, the polyclonal antibody can be prepared by making a complex of an immune antigen (antigen such as receptor protein and the like), immunizing a mammal as in the method for preparing the above monoclonal antibody, taking a material comprising an antibody to the present receptor protein from the immunized animal, and separating and purifying the antibody.

For the conjugate of the immune antigen with a carrier protein used for immunizing mammals, the type of the carrier protein and the mixing ratio of the carrier to the hapten are not particularly limited insofar as the desired antibody can be efficiently produced by immunization with the antigen crosslinked via the hapten with the carrier, and for example, the conjugate is produced by coupling the hapten with bovine serum albumin, bovine cyloglobulin, hemocyanin or the like in a ratio of 1 to about 0.1 to 20, preferably about 1 to 5.

The hapten can be coupled with the carrier by use of various condensing agents such as glutaraldehyde, carbodiimide, maleimide-activated ester, and activated ester reagents containing thiol group and dithiopyridyl group.

The resulting condensation product is administered alone or together with a carrier or a diluent into warm-blooded animals at a site where the antibody can be produced. To enhance the ability of the animals upon administration to produce the antibody, complete Freund's adjuvant or incomplete Freund's adjuvant may be administered. Administration is conducted usually once every 2 to 6 weeks and about 3 to 10 times in total.

The polyclonal antibody can be collected form blood, ascites etc. preferably blood in the mammals immunized by the method described above.

The polyclonal antibody titer in antiserum can be measured in the same manner as in the measurement of the antibody titer in antiserum as described above. Separation and purification of the polyclonal antibody can be carried out by a method of separating and purifying immunoglobulin, which is similar to the separation and purification of the monoclonal antibody as described above.

The present receptor protein or a salt thereof, a partial peptide thereof or a salt thereof, and a DNA encoding the receptor protein or a partial peptide thereof can be used for (1) determination of a ligand (agonist) for the present G protein coupled receptor protein, (2) an agent for preventing and/or treating diseases associated with the deficient function of the present G protein coupled receptor protein, (3) gene diagnostic agent, (4) a method for screening a compound which alters an amount of expression of the present receptor protein or a partial peptide thereof, (5) an agent for preventing and/or treating various diseases comprising a compound which alters an amount of expression of the present receptor protein or a partial peptide thereof, (6) a method for quantitating a ligand for the present G protein coupled receptor protein, (7) a method for screening a compound which alters binding of the present G protein coupled receptor protein with a ligand (agonist and antagonist), (8) an agent for preventing and/or treating various diseases comprising a compound which alters binding of the present G protein coupled receptor protein with a ligand (agonist and antagonist), (9) quantitation of the present receptor protein or a partial peptide thereof or a salt thereof, (10) a method for screening a compound which alters an amount of the present receptor protein or a partial peptide thereof in a cell membrane, (11) an agent for preventing and/or treating various diseases comprising a compound which alters an amount of the present receptor protein or its partial peptide in a cell membrane, (12) neutralization by an antibody to the present receptor protein or a partial peptide thereof or a salt thereof and (13) preparation of a non-human animal having a DNA encoding the present G protein coupled receptor protein.

In particular, by using a receptor binding assay system using an expression system of the present recombinant G protein coupled receptor protein, a compound which alters binding of a ligand with a G protein coupled receptor specific for a human being and a mammal (for example, agonist and antagonist) can be screened, the agonist or the antagonist can be used as an agent for preventing or treating various diseases.

The uses of the present receptor protein or a partial peptide thereof or a salt thereof (hereinafter abbreviated as present receptor protein and the like in some cases), a DNA encoding the present receptor protein or a partial peptide thereof (hereinafter abbreviated as present DNA in some cases) and an antibody to the present receptor protein and the like (hereinafter abbreviated as present antibody) will be explained specifically below.

### (1) Determination of a ligand (agonist) for the present G protein coupled receptor protein

The present receptor protein or a salt thereof or the present partial peptide or a salt thereof is useful as a reagent for searching or determining a ligand (agonist) for the present receptor protein or a salt thereof.

That is, the present invention provides a method for determining a ligand for the present receptor protein, which comprises contacting the present receptor protein or a salt thereof or a present partial peptide or a salt thereof, with a test compound.

As the test compound, the known ligands (for example, angiotensin, bombesin, canabinoide, cholecystokinin, glutamine, serotonin, melatonin, neuropeptide Y, opioid, purine, vasopressin, oxytocin, PACAP, secretin, glucagon, calcitonin, adrenomedulin, somatostatin, GHRH, CRF, ACTH, GRP, PTH, VIP (vasoactive intestinal polypeptide), somatostatin, dopamine, motilin, amyline, bradykinin, CGRP (calcitonin gene related peptide), leukotriene, pancreastatin, prostaglandin, thromboxane, adenosine, adrenaline, α and β-chemokine (for example, IL-8, GROα, GROβ, GRDγ, NAP-2, ENA-78, PF4, IP10, GCP-2, MCP-1, HC14, MCP-3, I-309, MIP1α, MIP-1β, RANTES), endothelin, enterogastrin, histamine, neurotensin, TRH, pancreatic polypeptide or galanin), as well as a tissue extract and a cell culture supernatant of a human being or a mammal (for example, mouse, rat, pig, cow, sheep and monkey) are used. For example, a single ligand can be finally obtained by adding the tissue extract or the cell culture supernatant to the present receptor protein, and fractionating while measuring the cell stimulating activity.

More particularly, a method for determining the present ligand is a method for determining a compound (for example, peptide, protein, non-peptide compound, synthetic compound and fermentation product) or a salt thereof having the cell stimulating activity (for example, the activity promoting or inhibiting arachidonic acid release, acetylcholine release, intracellular Ca²⁺ release, intracellular cAMP production, intracellular cGMP production, inositol phosphate production, cell membrane potential fluctuation, intracellular protein phosphorylation, c-fos activation, and reduction of pH) by binding with the present receptor protein, by using the present receptor protein or a partial peptide thereof or a salt thereof, or by using constructing an expression system for a recombinant receptor protein, and using a receptor finding assay system employing the expression system.

The method for determining a ligand of the present invention is characterized in that an amount of a test compound bound to the present receptor protein or a partial peptide and the cell stimulating activity when the receptor protein or the partial peptide thereof are contacted with a test compound are measured.

More particularly, the present invention provides:
(a) a method for determining a ligand for the present receptor protein or a salt thereof, which comprises measuring an amount of a labeled test compound bound to the present receptor protein or a salt thereof, or the present partial peptide or a salt in the case where a labeled test compound is contacted with the protein or a salt thereof or the partial peptide or a salt thereof,
(b) a method for determining a ligand for the present receptor protein or a salt thereof, which comprises measuring an amount of a labeled test compound bound to a cell comprising the present receptor protein or a membrane fraction of the cell in the case where a labeled test compound is contacted with the cell or the membrane fraction,
(c) a method for determining a ligand for the present receptor protein, which comprises measuring an amount of a labeled test compound bound to a receptor protein or a salt thereof in the case where a labeled test compound is contacted with the receptor protein expressed on a cell membrane by culturing a transformant comprising a DNA encoding the present receptor protein,
(d) a method for determining a ligand for the present receptor protein or a salt thereof, which comprises measuring the cell stimulating activity (for example, the activity promoting or inhibiting arachidonic acid release, acetylcholine release, intracellular Ca²⁺ release, intracellular cAMP production, intracellular cGMP production, inositol phosphate production, cell membrane potential fluctuation, intracellular protein phosphorylation, c-fos activation, and reduction of pH) via a receptor protein in the case where a test compound is contacted with a cell comprising the present receptor protein, and
(e) a method for determining a ligand for the present receptor protein or a salt thereof, which comprises measuring the cell stimulating activity (for example, the activity promoting or inhibiting arachidonic acid re lease, acetylcholine release, intracellular Ca²⁺ release, intracellular cAMP production, intracellular cGMP production, inositol phosphate production, cell membrane potential fluctuation, intracellular protein phosphorylation, c-fos activation, and reduction of pH) via a receptor protein in the case where a test compound is contacted with a receptor protein expressed on a cell membrane by culturing a transformant comprising a DNA encoding the present receptor protein.

In particular, it is preferable that the above experiments (d) to (e) are carried out after the above experiments (a) to (c) are carried out and binding of a test compound to the present receptor protein is confirmed.

First, as a receptor protein used for a ligand determining method, any receptor proteins may be used as long as they contain the aforementioned present receptor protein or present partial peptide and a receptor protein which was expressed at a large amount using an animal cell is suitable.

In order to prepare the present receptor protein, the aforementioned expression method is used but it is preferable that preparation is performed by expressing a DNA coding the receptor protein in a mammal cell or an insect cell. As a DNA fragment encoding a protein part of interest, a complementary DNA is usually used but is not necessarily limited to it. For example, a gene fragment or a synthetic DNA may be used. In order to introduce a DNA fragment encoding the present receptor protein into a host animal cell and express it effectively, it is preferable that the DNA fragment is incorporated into downstream of polyhedron promoter of nuclear polyhedrosis virus (NPV) belonging to Baculovirus for which a host is an insect, SV40 derived promoter, promoter of retrovirus, metallothionein promoter, human heat shock promoter, cytomegalovirus promoter and SRα promoter. Investigation of an amount and quality of the expressed receptor can be conducted by the per se known method. The investigation can be conducted by, for example, a method described in a reference [Nambi, P. et al., J. Biol. Chem., vol. 267, pp. 19555-19559, 1992].

Therefore, in the present ligand determining method, a material comprising the present receptor protein or a partial peptide thereof or a salt thereof may be a receptor protein or a partial peptide thereof or a salt thereof purified according to the per se known method, or a cell comprising the receptor protein or a cell membrane fraction thereof may be used.

In the present ligand determining method, when a cell comprising the present receptor protein is used, the cell may be fixed with glutaraldehyde or formalin. The fixing method can be conducted according to the per se known method.

A cell comprising the present receptor protein refers to a host cell which expressed the present receptor protein and, as the host cell, Escherichia coli, Bacillus subtilis, yeast, insect cell and animal cell are used.

A cell membrane fraction refers to a fraction in which a cell membrane obtained by the per se known method after rupture of a cell is contained at a large amount. As a method for breaking a cell, there are a method of squeezing a cell with a Potter-Elvehjem type homogenizer, breakage with a Waring Blender or Polytron (manufactured by Kinematica), breakage by ultrasound, and breakage by jetting a cell through a thin nozzle while pressuring with a French press. For fractionating a cell membrane, a fractionating method by centrifugation force such as a fractionating centrifugation method and density gradient centrifugation method is mainly used. For example, a broken cell solution is centrifuged at a low speed (500 rpm-3000 rpm) for shorter period of time (usually, about 1 minute to 10 minutes), the supernatant is centrifuged at a higher speed (15000 rpm-30000 rpm) usually for 30 minutes to 2 hours, and the resulting precipitate is used as a membrane fraction. An expressed receptor protein and membrane components such as cell-derived phospholipid and membrane protein are contained at a large amount in the membrane fraction.

An amount of a receptor protein in a cell comprising the receptor protein or its membrane fraction is preferably 10³-10⁸ molecules, suitably 10⁵-10⁷ molecules per cell. In addition, as an expressed amount is larger, ligand binding activity per a membrane fraction (specific activity) , not only high sensitive screening system can be constructed but also a large amount of samples can be measured at the same lot.

In order to conduct the above methods (a) to (c) for determining a ligand for the present receptor protein or a salt thereof, a suitable receptor protein fraction and a labeled test compound are necessary.

As a receptor protein fraction, a natural receptor protein fraction or a recombinant receptor fraction having the equal activity thereto are desirable. Here, equal activity denotes equal ligand binding activity and signal information transmission action.

As a labeled test compound, angiotensin, bombesin, canabinoide, cholecystokinin, glutamine, serotonin, melatonin, neuropeptide Y, opioid, purine, vasopressin, oxytocin, PACAP, secretin, glucagon, calcitonin, adrenomedulin, somatostatin, GHRH, CRF, ACTH, GRP, PTH, VIP (vasoactive intestinal polypeptide), somatostatin, dopamine, motilin, amyline, bradykinin, CGRP (calcitonin gene related peptide), leukotriene, pancreastatin, prostaglandin, thromboxane, adenosine, adrenaline, α and β-chemokine (for example, IL-8, GROα, GROβ, GROγ, NAP-2, ENA-78, PF4, IP10, GCP-2, MCP-1, HC14, MCP-3, I-309, MIP1α, MIP-1β, RANTES), endothelin, enterogastrin, histamine, neurotensin, TRH, pancreatic polypeptide and galanin which are labeled with [³H], [¹²⁵I], [¹⁴C] or [³⁵S] are suitable.

More particularly, in order to conduct a method for determining a ligand for the present receptor protein or a salt thereof, first, an authentic receptor is prepared by suspending a cell comprising the present receptor protein or a membrane fraction of the cell in a buffer suitable for the determination method. Any buffers which do not inhibit binding of a ligand with a receptor protein may be used such as phosphate buffer and Tris-hydrochloric acid buffer of pH 4-10 (desirably pH 6-8). In addition, in order to decrease non-specific binding, surfactants such as CHAPS, Tween-80™ (Kao-Atlas Company), digitonin, and deoxycholate and various proteins such as bovine serum albumin and gelatin may be added to a buffer. Further, in order to suppresss degradation of a receptor and a ligand by a protease, a protease inhibiting agent such as PMSF, leupeptin, E-64 (manufactured by Peptide Laboratory) and pepstain may be added. A test compound labeled with a constant amount (5000 cpm-500000 cpm) of [³H], [¹²⁵I], [¹⁴C] and [³⁵S] is present in 0.01 ml to 10 ml of the receptor solution. In order to know an amount of non-specific binding (NSB), a reaction tube to which a largely excessive amount of an unlabeled test compound has been added is also prepared. The reaction is conducted at about 0 °C to 50 °C, preferably about 4 °C to 37 °C, for about 20 minutes to 24 hours, desirably about 30 minutes to 3 hours. After the reaction, the reaction is filtered with a glass fiber filtering paper, washed with a suitable amount of the same buffer, and the radioactivity remaining in a glass fiber filtering paper is measured by a scintillation counter or a γ-counter. A test compound having greater than zero cpm of a count (B-NSB) obtained by a total binding amount (B) minus a non-specific binding amount (NSB) can be selected as a ligand (agonist) for the present receptor protein or a salt thereof.

In order to conduct the above methods (d)-(e) for determining a ligand for the present receptor protein or a salt thereof, the cell stimulating activity (for example, the activity promoting or inhibiting arachidonic acid release, acetylcholine release, intracellular Ca²⁺ release, intracellular cAMP production, intracellular cGMP production, inositol phosphate production, cell membrane potential fluctuation, intracellular protein phosphorylation, c-fos activation, and reduction of pH) via the receptor protein can be measured by the known method or using a commercially available measuring kit. More particularly, first, a cell comprising a receptor protein is cultured on a multiwellplate. Upon implementation of ligand determination, a medium and a buffer are exchanged with a fresh medium or a suitable buffer showing no toxicity to a cell in advance, a test compound is added to incubate for a constant period of time, a cell is extracted or the supernatant is recovered, and the produced product is quantitated according to respective methods. When the production of a substance as an index for the cell stimulating activity (for example, arachidonic acid) is difficult to be assayed due to a degrading enzyme contained in a cell, an assay may be conducted by adding an inhibitor for the degrading enzyme. In addition, regarding the activity such as cAMP production inhibition, it can be detected as production inhibiting action to a cell for which a fundamental producing amount of a cell has been increased by froscolin.

A kit for determining a ligand which binds to the present receptor protein or a salt thereof comprises the present receptor protein or a salt thereof, the present partial peptide or a salt thereof, a cell comprising the present receptor protein, or a membrane fraction of a cell comprising the present receptor protein.

Examples of the present ligand determining kit are as follows.
1. Ligand determining reagent
   (a) A measuring buffer and a washing buffer
      Hanks' Balanced Salt Solution (manufactured by Gibco) with 0.05% bovine serum albumin (manufactured by Sigma) added
      Buffers may be sterile-filtered with a filter having a pore diameter of 0.45 µm and stored at 4 °C, or may be prepared upon use.
   (b) Authentic G protein coupled receptor protein
      CHO cells which expressed the present receptor protein are passaged into a 12-well plate at 5 × 10⁵ well, and cultured at 37°C and 5% CO₂ and 95% air for 2 days.
   (c) Labeled test compound
      A compound labeled with commercially available [³H], [¹²⁵I], [¹⁴C] or [³⁵S], or labeled with a suitable method
      An aqueous solution is stored at 4 °C or -20 °C, and diluted with a measuring buffer to 1 µM upon use. A test compound showing poor solubility in water is dissolved in dimethylformamide, DMSO or methanol.
   (d) Unlabeled test compound
      The same compound as labeled compound is prepared at the 100- to 1000-fold concentration.
2. Measuring method
   (a) CHO cells expressing the present receptor protein cultured by a 12-well tissue culturing plate are washed twice with 1 ml of a measuring buffer, and 490 µl measuring buffer is added to each well.
   (b) 5 µl of a labeled test compound is added to react at room temperature for 1 hour. In order to know an amount of non-specific binding, 5 µl of a non-labeled test compound is added.
   (c) A reaction solution is removed and washed three times with 1 ml of a washing buffer. A labeled test compound bound to a cell is dissolved in 0.2 N NaOH-1% SDS, and mixed with 4 ml of a liquid scintillator A (manufactured by Wako Pure Chemical Industries, Ltd.).
   (d) The radioactivity is measured using a liquid scitillation counter (manufactured by Beckmann).

As a ligand which can bind to the present receptor or a salt thereof, for example, there are substances present in brain, pituitary gland and pancreas, and more particularly, angiotensin, bombesin, canabinoide, cholecystokinin, glutamine, serotonin, melatonin, neuropeptide Y, opioid, purine, vasopressin, oxytocin, PACAP, secretin, glucagon, calcitonin, adrenomedulin, somatostatin, GHRH, CRF, ACTH, GRP, PTH, VIP (vasoactive intestinal polypeptide), somatostatin, dopamine, motilin, amyline, bradykinin, CGRP (calcitonin gene related peptide), leukotriene, pancreastatin, prostaglandin, thromboxane, adenosine, adrenaline, α and β-chemokine (for example, IL-8, GROα, GROβ, GROγ, NAP-2, ENA-78, PF4, IP10, GCP-2, MCP-1, HC14, MCP-3, I-309, MIP1α, MIP-1β, RANTES), endothelin, enterogastrin, histamine, neurotensin, TRH, pancreatic polypeptide and galanin are used.

### (2) An agent for preventing and/or treating diseases associated with the deficient of the present G protein coupled receptor protein

In the above (1) method, when a ligand for the present receptor protein is revealed, (a) the present receptor protein or (b) a DNA encoding the receptor protein can be used as a medicine for preventing and/or treating diseases associated with the deficient function of the present receptor protein depending upon the action harbored by the ligand.

For example, when there is a patient for whom the ligand physiological action can not be expected (lack of the receptor protein) due to a decrease in the present receptor protein in the living body, an amount of a receptor protein in the living body of the patient can be increased and the action of a ligand can be sufficiently exerted by (i) compensating for an amount of the receptor protein by administering the present receptor protein to the patient or by (ii) (a) administering a DNA encoding the present receptor protein to the patient to express the DNA, or (b) inserting a DNA encoding the present receptor protein into a subject cell to express the DNA, and transplanting the cell into the patient. That is, a DNA encoding the present receptor protein is useful as a safe and low toxic agent for preventing and/or treating diseases associated with the deficient function of the present receptor protein.

The present receptor protein is recognized to have about 30% homology with angiotensin II type I receptor (AT1) which is a G protein coupled receptor protein at an amino acid sequence level. AT1 is reported to be associated with the pathology of hypertension, hypercardia and arterial sclerosis (Nippon Clinics, vol. 56, No. 7, 1906-1911 (1998)). Therefore, the present receptor having the recognized homology with AT1 is useful for preventing and/or treating circulatory diseases (for example, hypertension, hypercardia and arterial sclerosis). In addition, as the actions via ATT1, the actions of vasopression/LH/FSH secretion, water uptake, sodium cloride preference, and automatical regulaion of brain blood stream in the central, hypertrophy, constriction enhancing and remodeling in a heart, aldosteron secresion from adrenal cortex, catecholamine secresion from adrenal medulla, promotion of resorbence of sodium in a proximal uriniferous tubule, constriction of an exporting/importing arteriole in a kidney, constriction and hypertrophy promotion in a blood vessel are known [Current Neural Endocrinology (supervised by Sho Yoshida), pp.75-83). Therefore, the present receptor protein is useful for preventing and/or treating respiratory diseases, circulatory diseases, digestive tract diseases, liver/gallbladder/pancreas diseases, and endocrine diseases.

When the present receptor protein is used as the above preventing or treating agent, it can be formulated according to the conventional means.

On the other hand, when a DNA encoding the present receptor protein (hereinafter abbreviated as present DNA in some cases) is used as the above preventing or treating agent, the present DNA can be implemented according to the conventional means, alone or after inserted into a suitable vector such as a retrovirus vector, an adenovirus vector, and an adenovirus vector-associated virus vector. The present DNA can be administered alone or together with an auxiliary agent for promoting uptake by a catheter such as a gene gun or a hydrogel.

For example, (a) the present receptor protein or (b) a DNA encoding the receptor protein can be used orally as tablets which is coated with a sugar-coating if necessary, capsules, elixirs, or microcapsules, or can be used parenterally in the form of an injection such as a sterile solution or a suspension with water or other pharmaceutically acceptable solution. For example, (a) the present receptor protein or (b) a DNA encoding the receptor protein can be prepared by blending with a physiologically acceptable carriers, flavors, excipients, vehicles, preservatives, stabilizers and binders into an unit dosage form required for the generally recognized preparation implementation. An amount of an effective ingredient in these preparations is such that a suitable volume in an indicated range can be obtained.

The additives which can be admixed with the tablets, capsules etc. include, for example, binders such as gelatin, corn starch, tragacanth, gum arabic, excipients such as crystalline cellulose, swelling agents-such as corn starch, gelatin and alginic acid, lubricants such as magnesium stearate, sweeteners such as sucrose, lactose and saccharine, and flavors such as peppermint, akamono oil and cherry. When one capsule is in a unit form, liquid carriers such as fats and oils can be contained in the materials described above. The aseptic composition for injection can be formulated according to conventional pharmaceutical manufacturing by dissolving or suspending the active material and naturally occurring vegetable oils such as sesame oil and coconut oil in vehicles such as injection water. The aqueous solution for injection includes, for example, physiological saline or an isotonic solution containing glucose and other supplementary agents (e.g., D-sorbitol, D-mannitol, sodium chloride etc.), and may be used in combination with suitable solubilizer such as alcohols (e.g., ethanol etc.), polyalcohols (e.g., propylene glycol, polyethylene glycol etc.) and nonionic surfactants (e.g., Polysorbate 80™, HCO-50 etc.). The oily solution includes, for example, sesame oil, soybean oil etc., and may be used in combination with solubilizer such as benzyl benzoate, benzyl alcohol etc. In addition, the above preventing or treating agent may be blended, for example, with the buffer (e.g., phosphate buffer, sodium acetate buffer etc.), soothing agents (e.g., benzalkonium chloride, procaine hydrochloride etc.), stabilizers (e.g., human serum albumin, polyethylene glycol etc.), preservatives (e.g., benzyl alcohol, phenol etc.), antioxidants etc. Usually, the prepared injection is introduced into suitable ampoules.

Since the thus obtained preparation is safe and low toxic, it can be administered, for example, to a human being or a mammal (such as rat, mouse, rabbit, sheep, cow, cat, dog, and monkey).

A dose of the present receptor protein is different depending upon an administration subject, a subject organ, sympton and a route of administration. When orally administered, the dose is generally about 0.1 mg to 100 mg, preferably about 1.0 to 50 mg, more preferably about 1.0 to 20 mg per day in a hypertension patient (60 kg). When parenterally administered, one time dose is different depending upon an administration subject, a subject organ, sympton and an administration method and, for example, in the form of an injectable, it is around about 0.01 to 30 mg, preferably around about 0.1 to 20 mg, more preferably around about 0.1 to 10 mg per day by intravenous injection in a hypertension patient (60 kg). In the case of other animal, an amount calculated per 60 kg can be administered.

A dose of the present DNA is different depending upon an administration subject, a subject organ, sympton and an administration methpd and, for example, in the case of oral administration, it is generally about 0.1 mg to 100 mg, preferably about 1.0 to 50 mg, more preferably about 1.0 to 20 mg per day in a hypertension patient (60 kg). When parenterally administered, one time dose is different depending upon an administration subject, a subject organ, sympton and an administration method and, for example, in the form of an injection, it is advantageous to administer around about 0.01 to 30 mg, preferably around about 0.1 to 20 mg, more preferably around about 0.1 to 10 mg per day in a hypertension patient. In the case of other animals, an amount calculated per 60 kg can be administeed.

### (3) Gene diagnostic agent

Since the present DNA can detect abnormality of a DNA or a mRNA encoding the present receptor or a partial peptide thereof (gene abnormality) in a human being or a mammal (such as rat, mouse, rabbit, sheep, pig, cow, cat, dog and monkey) by using as a probe, it is useful as a gene diagnostic agent for damage, mutation or expression reduction of the DNA or mRNA, or increase or excess expression of the DNA or mRNA.

The above gene diagnosis using the present DNA can be conducted, for example, by the per se known method of Northern hybridization or PCR-SSCP (Genomics, vol. 5, pp.874-879 (1989), Proceeedings of the National Academy of Sciences of the United States of America, vol.86, pp. 2766-2770 (1989)).

### (4) A method for screening a compound which alters an amount of expression of the present receptor protein or a partial peptide thereof

The present DNA can be used for screening a compound which alters an amount of expression of the present receptor protein or a partial peptide thereof by using as a probe.

That is, the present invention provides a method for screening a compound which alters an amount of expression of the present receptor protein or a partial peptide thereof, by measuring an amount of a mRNA of the present receptor or a partial peptide thereof contained in (i) non-human mammal-derived (a) blood, (b) particular organ, and (c) tissue or cell isolated from an organ, or (ii) transformant.

Measurement of an amount of a mRNA of the present receptor protein or a partial peptide thereof is specifically conducted as follows.
(i) A drug (for example, anti-dementia drug, blood pressure decreasing agent, anti-cancer agent and anti-obesity drug) or a physical stress (for example, inundation stress, electric stress, light and dark, low temperature) is given to a normal or disease model non-human mammal (for example, mouse, rat, rabbit, sheep, pig, cow, cat, dog and monkey, more particularly, dementiarat, obesity mouse, arterial sclerosis rabbit and cancer carrying mouse) and, after a predetermined period of time, blood or particular organs (for example, brain, liver and kidney), or a tissue or a cell isolated from organs is obtained.
   A mRNA of the present receptor protein or a partial peptide thereof contained in the resulting cell can be quantitated, for example, by extracting a mRNA from a cell and the like by a normal method and, for example, using a method such as TaqManPCR and can be also analyzed by conducting Northern blot by the per se known means.
(ii) A transformant expressing the present receptor protein or a partial peptide is prepared according to the aforementioned method, a mRNA of the present receptor protein or a partial peptide thereof contained in the transformant can be quantitated and analyzed similarly.

Screening of a compound which alters an amount of expression of the present receptor protein or a partial peptide thereof can be conducted by
(i) administering a test compound to a normal or a disease model non-human mammal, a constant period of time before (30 minutes to 24 hours before, preferably 30 minutes to 12 hours before, more preferably 1 hour to 6 hours before), or a constant period of time after (30 minutes to 3 days after, preferably 1 hour to 2 days after, more preferably 1 hour to 24 hours after) giving a drug or a physical stress, or at the same time with a drug or a physical stress, and quantitating and analyzing an amount of a mRNA of the present receptor protein or a partial peptide contained in a cell after a constant period of time (30 minutes to 3 days, preferably 1 hour to 2 days, more preferably 1 hour to 24 hours) has passed, and by
(ii) mixing a test compound into a medium upon culturing of a transformant according to the conventional method, and quantitating and analyzing an amount of a mRNA of the present receptor protein or a partial peptide thereof contained in the transformant after a constant period of time of culturing (after 1 day to 7 days, preferably after 1 day to 3 days, more preferably after 2 days to 3 days).

A compound or a salt thereof obtained by using the present screening method is a compound having the activity which alters an amount of expression of the present receptor protein or a partial peptide thereof, more particularly, (a) a compound which enhances the cell stimulating activity (for example, theactivity promoting or inhibiting arachidonic acid release, acetylcholine release, intracellular Ca²⁺ release, intracellular cAMP production, intracellular cGMP production, inositol phosphate production, cell membrane potential fluctuation, intracellular protein phosphorylation, c-fos activation, and reduction of pH) via a G protein coupled receptor by increasing an amount of expression of the present receptor protein or a partial peptide thereof, (b) a compound which reduces the cell stimulating activity by decreasing an amount of expression of the present receptor protein or a partial peptide thereof.

Examples of the compound include a peptide, a protein, a non-peptide compound, a synthetic compound and a fermentation product. Theses compounds may be a novel compound or the known compound.

A compound which enhances the cell stimulating activity is useful as a safe and low toxic medicine for enhancing the physiological activity of the present receptor protein and the like.

A compound which reduces the cell stimulating activity is useful as a safe and low toxic medicine for decreasing the physiological activity of the present receptor protein and the like.

When a compound or a salt thereof obtained by using the present screening method is used as a pharmaceutical composition, the use can be implemented according to the conventional means. For example, the compound may be formulated into tablets, capsules, elixirs, microcapsules, sterile solution and suspension as in the aforementioned medicine comprising the present receptor protein.

Since the thus obtained preparations are safe and low toxic, they can be administered, for example, to a human being or a mammal (for example, rat, mouse, rabbit, sheep, pig, cow, cat, dog and monkey).

A dose of the compound or a salt thereof is different depending upon an administration subject, a subject organ, sympton and an administration method and, when orally administered, the dose is generally about 0.1 to 100 mg, preferably about 1.0 to 50 mg, more preferably about 1.0 to 20 mg per day in a hypertension patient (60 kg). When parenterally administered, one time dose is different depending upon an administration subject, a subject organ, sympton, and an administration method and it is usually advantageous administer at an amount of around about 0.01 to 30 mg, preferably around about 0.1 to 20 mg, more preferably around about 0.1 to 10 mg per day by intravenous injection in the form of an injectable in a hypertension patient (60 kg). In the case of other animals, an amount calculated per 60 kg can be administered.

### (5) An agent for preventing and/or treating various diseases comprising a compound which alters an amount of expression of the present receptor protein or a partial peptide thereof

It is considered that the present receptor protein plays some important role in the living body such as central functions as described above. Therefore, a compound which alters an amount of expression of the present receptor protein or a partial peptide thereof can be used as an agent for preventing and/or treating diseases associated with the deficient functions of the present receptor protein.

When the compound is used as an agent for preventing and/or treating diseases associated with the deficient functions of the present receptor protein, it can be formulated according to the conventional means.

For example, the compound can be used orally as tablets coated with a sugar-coating if necessary, capsules, elixirs and microcapsules, or parenterally in the form of an injection such as a sterile solution with water or other pharmaceutically acceptable solution, or suspension. For example, the preparations may be produced by mixing the compound with the physiologically recognized known carriers, flavors, excipients, vehicles, antiseptics, stabilizers and binders in an unit dosage form required for generally recognized preparation implement. An amount of an active ingredient in these preparations is such that a suitable volume in an indicated range is obtained.

The additives which can be admixed with the tablets, capsules etc. include, for example, binders such as gelatin, corn starch, tragacanth, gum arabic, excipients such as crystalline cellulose, swelling agents such as corn starch, gelatin and alginic acid, lubricants such as magnesium stearate, sweeteners such as sucrose, lactose and saccharine, and flavors such as peppermint, akamono oil and cherry. When one capsule is in a unit form, liquid carriers such as fats and oils can be contained in the materials described above. The aseptic composition for injection can be formulated according to conventional pharmaceutical manufacturing by dissolving or suspending the active material and naturally occurring vegetable oils such as sesame oil and coconut oil in vehicles such as injection water. The aqueous solution for injection includes, for example, physiological saline or an isotonic solution containing glucose and other supplementary agents (e.g. D-sorbitol, D-mannitol, sodium chloride etc.), and may be used in combination with suitable solubilizer such as alcohols (e.g., ethanol etc.), polyalcohols (e.g., propylene glycol, polyethylene glycol etc.) and nonionic surfactants (e.g., Polysorbate 80™, HCO-50 etc.). The oily solution includes, for example, sesame oil, soybean oil etc., and may be used in combination with solubilizer such as benzyl benzoate, benzyl alcohol etc. In addition, the above preventing or treating agent may be blended, for example, with the buffer (e.g., phosphate buffer, sodium acetate buffer etc.), soothing agents (e.g., benzalkonium chloride, procaine hydrochloride etc.), stabilizers (e.g., human serum albumin, polyethylene glycol . etc.), preservatives (e.g., benzyl alcohol, phenol etc.), antioxidants etc. Usually, the prepared injection is introduced into suitable ampoules.

Since the thus obtained preparations are safe and low toxic, they may be administered, for example, to a human being or a mammal (for example, rat, mouse, rabbit, sheep, pig, cow, cat, dog and monkey).

A dose of the compound or a salt thereof is different depending upon an administration subject, a subject organ, sympton and an administration method and, when orally administered, the dose is generally about 0.1 to 100 mg, preferably about 1.0 to 50 mg, more preferable about 1.0 to 20 mg per day in a hypertention patient (60 kg). When parenterally administered, one time dose is different depending upon an administration subject, a subject organ, sympton and an administration method and it is usually advantageous to administer by intravenous injection at an amount of around about 0.01 to 30 mg, preferably around about 0.1 to 20 mg, more preferably around about 0.1 to 10 mg per day in the form of an injectable in a hypertention patient (60 kg). In the case of other animals, an amount calculated per 60 kg can be administered.

### (6) A method for quantitating a ligand for the present G protein coupled receptor protein

Since the present receptor protein and the like have the property of binding to a ligand, the ligand concentration in the living body can be quantitated with a better sensitivity.

The present quantitation method can be used by combining, for example, with a competition method. That is, the ligand concentration in a test compound can be measured by contacting a test compound with the present receptor protein and the like. More particularly, for example, the method can be used according to a method described in the following (a) or (b) or its analogous method.
(a) "Radioimmunoassay" ed. by Hiroshi Irie (Kodansha, published in 1974)
(b) "Radioimmunoassay", "second series" ed. by Hiroshi Irie (Kodansha, published in 1979)
(g) A method for screening a compound (such as agonist and antagonist) which alters binding of the present G protein coupled receptor protein with a ligand

A compound (for example, peptide, protein, non-peptide compound, synthetic compound, and fermentation product) or a salt thereof can be effectively screened by using the present receptor protein and the like, or constructing an expression system for a recombinant receptor protein and using a receptor binding assay system using the expression system.

Such the compound includes (a) a compound having the cell stimulating activity (for example, the activity promoting or inhibiting arachidonic acid release, acetylcholine release, intracellular Ca²⁺ release, intracellular cAMP production, intracellular cGMP production, inositol phosphate production, cell membrane potential fluctuation, intracellular protein phosphorylation, c-fos activation, and reduction of pH) via a G protein coupled receptor (so called agonist for the present receptor protein), (b) a compound having no cell stimulating activity (so called antagonist for the present receptor protein), (c) a compound which enhances binding of a ligand with the present G protein coupled receptor protein, or (d) a compound which reduces binding of a ligand with the present G protein coupled receptor protein (for the above (a) compound, it is preferable to screen by the aforementioned ligand determining method).

That is, the present invention provides a method for screening a compound or a salt thereof which alters binding of a ligand with the present receptor protein or a partial peptide thereof or a salt thereof, which comprises comparing (i) the case where the present receptor protein or a partial peptide thereof or a salt thereof is contacted with a ligand, with (ii) the case where the present receptor protein or a partial peptide thereof or a salt thereof is contacted with a ligand and a test compound.

The present screening method is characterized in that, for example, an amount of a ligand bound to the receptor protein and the like, and the cell stimulating activity in the case of (i) and (ii) are measured and compared.

More particularly, the present invention provides:
(a) a method for screening a compound or a salt thereof which alters binding of a ligand with the present receptor protein and the like, which comprises measuring and comparing an amount of a labeled ligand bound to the receptor protein and the like in the case where a labeled ligand is contacted with the present receptor protein and the like and the case where a labeled ligand and a test compound are contacted with the present receptor protein and the like,
(b) a method for screening a compound or a salt thereof which alters binding of a ligandwith the present receptor protein and the like, which comprises measuring and comparing an amount of a labeled ligand bound to a cell comprising the present receptor protein and the like or a membrane fraction of the cell in the case where a labeled ligand is contacted with the cell or the membrane fraction and the case where a labeled ligand and a test compound are contacted with the cell or the membrane fraction,
(c) a method for screening a compound or a salt thereof which alters binding of a ligandwith the present receptor protein and the like, which comprises measuring and comparing an amount of a labeled ligand bound to a receptor protein and the like expressed on a cell membrane by culturing a transformant comprising the present DNA in the case where a labeled ligand is contacted with the receptor protein and the like and the case where a labeled ligand and a test compound are contacted with the receptor protein and the like,
(d) a method for screening a compound or a salt thereof which alters binding of a ligandwith the present receptor protein and the like, which comprises measuring and comparing the cell stimulating activity (for example, the activity promoting or inhibiting arachidonic acid release, acetylcholine release, intracellular Ca²⁺ release, intracellular cAMP production, intracellular cGMP production, inositol phosphate production, cell membrane potential fluctuation, intracellular protein phosphorylation, c-fos activation, and reduction of pH) via a receptor in the case where a compound which activates the present receptor protein and the like (for example, a ligand for the present receptor protein and the like) is contacted with a cell comprising the present receptor protein and the like and the case where a compound which activates the present receptor protein and the like and a test compound are contacted with a cell comprising the present receptor protein and the like, and
(e) a method for screening a compound a salt thereof which alters binding of a ligand with the present receptor protein and the like, which comprises measuring and comparing the cell stimulating activity (for example, the activity promoting or inhibiting arachidonic acid release, acetylcholine release, intracellular Ca²⁺ release, intracellular CAMP production, intracellular cGMP production, inositol phosphate production, cell membrane potential fluctuation, intracellular protein phosphorylation, c-fos activation, and reduction of pH) via a receptor in the case where a compound which activates the present receptor protein and the like (for example, a ligand for the present receptor protein and the like) is contacted with the present receptor protein and the like expressed on a cell membrane by culturing a transformant comprising the present DNA and the case where a compound which activates the present receptor protein and the like and a test compound are contacted with the present receptor protein and the like expressed on a cell membrane by culturing a transformant comprising the present DNA.

Before the present receptor protein and the like are obtained, when a G protein coupled receptor agonist or antagonist is screened, it was necessary to obtain first a candidate compound using a cell, a tissue or its cell membrane fraction comprising a G protein coupled receptor protein of a rat (primary screening) and, thereafter, to confirm whether the candidate compound actually inhibits binding of a human G protein coupled receptor protein with a ligand (secondary screening). When a cell, a tissue or a cell membrane fraction is used as it is, since other receptor proteins are present in admixture therewith, it was difficult to actually screen an agonist or an antagonist for a receptor protein of interest.

However, for example, the use of the present human-derived receptor protein eliminates the necessity of the primary screening and can effectively screen a compound which inhibits binding of a ligand with a G protein coupled receptor. Further, whether the screened compound is an agonist or an antagonist can be simply assessed.

The present screening method will be specifically explained in detail.

First, although as the present receptor protein and the like used for the present screening method, any receptor proteins comprising the aforementioned present receptor protein and the like may be used, a cell membrane fraction of an organ of a mammal comprising the present receptor protein and the like is suitable. However, since it is extremely difficult to obtain, in particular, a human-derived organ, a human-derived receptor protein and the like expressed at a large amount using a transformant are suitable for use in screening.

For preparing the present receptor protein and the like, the aforementioned method is used but it is preferable to prepare by expression of the present DNA in a mammal cell or an insect cell. As a DNA fragment encoding a protein part of interest, a complementary DNA is used but is not necessarily limited to it. For example, a gene fragment for a synthetic DNA may be used. In order to introduce a DNA fragment encoding the present receptor protein into a host animal cell and express it effectively, it is preferable to incorporate the DNA fragment downs tream of a polyhedrin promoter of nuclear polyhedrosis virus (NPV) belonging to Baculovirus for which a host is an insect, a SV40 derived promoter, a retrovirus promoter, a metallothionein promoter, a human heat shock promoter, a cytomegalovirus promoter and a SRα promoter. Investigation of amount and quality of the expressed receptor can be carried out by the per se known method. It can be carried out, for example, by a method described in the reference [Nambi, P. et al., J. Biol. Chem., vol. 267, pp. 19555-19559, 1992].

Therefore, in the present screening method, a material comprising the present receptor protein and the like may be a receptor protein and the like purified according to the per se known method, a cell comprising the receptor protein and the like may be used, or a membrane fraction of a cell comprising the present protein and the like may be used.

In the present screening method, when a cell comprising the present receptor protein and the like is used, the cell may be fixed with glutaraldehyde or formalin. A fixing can be conducted according to the per se known method.

A cell comprising the present receptor protein and the like refers to a host cell which has expressed the receptorprotein and the like and, as the host cell, Escherichia coli, Bacillus subtilis, yeast, insect cell and animal cell are preferable.

A cell membrane fraction refers to a fraction comprising a large amount of a cell membrane obtained by the per se known method after rupture of a cell. As a method for breaking a cell, there are a method of squeezing a cell with a Potter-Elvehjem type homogenizer, breakage with a Waring Blender or Polytron (manufactured by Kinematica), breakage by ultrasound, and breakage by jetting a cell through a thin nozzle while pressuring with a French press. For fractionating a cell membrane, a fractionating method by centrifugation force such as a fractionating centrifugation method and density gradient centrifugation method is mainly used. For example, a broken cell solution is centrifuged at a low speed (500 rpm-3000 rpm) for a shorter period of time (usually, about 1 minute to 10 minutes), the supernatant is centrifuged at a higher speed (15000 rpm-30000 rpm) usually for 30 minutes to 2 hours, and the resulting precipitate is used as a membrane fraction. An expressed receptor protein and membrane components such as cell-derived phospholipid and a membrane protein are contained at a large amount in the membrane fraction.

An amount of a receptor protein in a cell comprising the receptor protein or a membrane fraction is preferably 10³-10⁸ molecules, suitably 10⁵-10⁷ molecules per cell. In addition, as an expressed amount is larger, ligand binding activity per a membrane fraction (specific activity) becomes higher, and not only high sensitive screening system can be constructed but also a large amount of samples can be measured at the same lot.

In order to conduct the above methods (a) to (c) for screening a compound which alters binding of a ligand with the present receptor protein and the like, a suitable receptor protein fraction and a labeled ligand are necessary.

As a receptor protein fraction, a natural receptor protein fraction or a recombinant receptor protein fraction having the equal activity thereto are desirable. Here, equal activity denotes equal ligand binding activity and signal information transmission action.

As a labeled ligand, a labeled ligand and a labeled ligand analog compound are used. For example, a ligand labeled with [³H], [¹²⁵I], [¹⁴C] or [³⁵S] is used.

More particularly, in order to conduct a method for screening a compound which alters binding of a ligand with the present receptor protein and the like, first, an authentic receptor protein is prepared by suspending a cell comprising the present receptor protein and the like or a membrane fraction of the cell in a buffer suitable for screening. Any buffers which do not inhibit binding of a ligand with a receptor protein may be used such as phosphate buffer and Tris-hydrochloric acid buffer of pH 4-10 (desirably pH 6-8). In addition, in order to decrease non-specific binding, surfactants such as CHAPS, Tween-80™ (Kao-Atlas Company) , digitonin, and deoxycholate may be added to a buffer. Further, in order to suppress degradation of a receptor and a ligand by a protease, a protease inhibiting agent such as PMSF, leupeptin, E-64 (manufactured by Peptide Laboratory) and pepstatin may be added. A ligand labeled with a constant amount (5000 cpm-500000 cpm) is added to 0.01 ml to 10 ml of the receptor solution and 10⁻⁴ M to 10⁻¹⁰ M of a test compound is present at the same time. In order to know an amount of non-specific binding (NSB), a reaction tube to which a largely excessive amount of an unlabeled test compound has been added is also prepared. The reaction is conducted at about 0 °C to 50 °C, preferably about 4 °C to 37 °C, for about 20 minutes to 24 hours, desirably about 30 minutes to 3 hours. After the reaction, the reaction is filtered with a glass fiber filtering paper, washed with a suitable amount of the same buffer, and the radioactivity remaining in a glass fiber filtering paper is measured by a scintillation counter or a γ-counter. A test compound having, for example, not greater than 50% of a specific binding amount (B-NSB) when a count (B₀-NSB) obtained subtracting a non-specific binding amount (NSB) from a count (B₀) at no antagonizing substance is regarded as 100%, can be selected a candidate compound having the antagonism inhibiting ability.

In order to conduct the above methods (d) - (e) for screening a compound which alters binding of a ligand with the present protein and the like, for example, the cell stimulating activity (for example, the activity promoting or inhibiting arachidonic acid release, acetylcholine release, intracellular Ca²⁺ release, intracellular cAMP production, intracellular cGMP production, inositol phosphate production, cell membrane potential fluctuation, intracellular protein phosphorylation, c-fos activation, and reduction of pH) via a receptor protein can be measured by the known method or using a commercially available measuring kit.

More particularly, first, a cell comprising the present receptor protein and the like is cultured on a multiwellplate. Upon implementation of screening, a medium and a buffer are exchanged with a fresh medium or a suitable buffer showing no toxicity to a cell in advance, a test compound is added to incubate for a constant period of time, a cell is extracted or the supernatant is recovered, and the produced product is quantitated according to respective methods. When the production of a substance as an index for the cell stimulating activity (for example, arachidonic acid) is difficult to be assayed due to a degrading enzyme contained in a cell, an assay may be conducted by adding an inhibitor for the degrading enzyme. In addition, regarding the activity such as cAMP production inhibition, it can be detected as production inhibiting action to a cell for which a fundamental producing amount of a cell has been increased by froscolin.

In order to conduct screening by measuring the cell stimulating activity, a cell which has expressed a suitable receptor protein is necessary. As a cell which has expressed the present receptor protein, a cell strain having natural type present receptor protein and the like, and a cell which has expressed the aforementioned recombinant receptor protein and the like are desirable.

As a test compound, for example, a peptide, a protein, a non-peptide compound, a synthetic compound, a fermentation product, a cell extract, a plant extract and an animal tissue extract are used and these compounds may be a novel compound or the known compound.

A kit for screening a compound which alters binding of a ligand with the present receptor protein and the like, or a salt, contains a cell comprising the present receptor protein and the like, or a membrane fraction of a cell comprising the present receptor protein and the like.

An example of the present kit for screening is as follows:
1. Screening reagent
   (a) Measuring buffer and washing buffer
      Hanks' Balanced Salt Solution (manufactured by Gibco) with 0.05% bovine serum albumin (manufactured by Sigma) added.
      The buffers may be sterile filtered with a filter of a pore size of 0.45 µm and stored at 4 °C, or may be prepared upon use.
   (b) Authentic G protein coupled receptor
      CHO cells which have expressed the present receptorprotein are passaged in a 12-well plate at 8 × 10⁵/well and cultured at 37 °C and 5% Co₂ and 95% air for 2 days.
   (c) Labeled ligand
      Ligand labeled with commercially available [³H], [¹²⁵I], [¹⁴C] or [³⁵S]. The ligand in the state of an aqueous solution is stored at 4 °C or -20 °C, and diluted with a measuring buffer to 1 µM upon use.
   (d) Ligand standard solution
      A ligand is dissolved in PBS comprising 0.1% bovine serum albumin (manufactured by Sigma) to 1 mM and stored at -20 °C.
2. A measuring method
   (a) CHO cells, expressing the present receptor protein, which were cultured on a 12-well tissue culturing plate, are washed with twice with 1 ml of a measuring buffer, and 490 µl measuring buffer is added to each well.
   (b) 5 µl of a 10⁻³ to 10⁻¹⁰ M test compound is added, and 5 µl of a labeled ligand is added to react at room temperature for 1 hour. In order to know an amount of non-specific binding, 5 µl of a 10⁻³ M ligand is added in place of a test compound.
   (c) The reaction solution is removed, and washed three times with 1 ml of a washing buffer. A labeled ligand bound to a cell is dissolved in 0.2N NaOH-1% SDS, and mixed with 4 ml of a liquid scintillator A (manufactured by Wako Pure Chemical Industries, Ltd.).
   (d) The radioactivity is measured using a liquid scintillation counter (manufactured by Beckmann) and Percent Maximum Binding (PMB) is obtained according to the following equation.
      PMB= [(B-NSB)/(B₀-NSB)] ×100
      PMB: Percent Maximum Binding
      B: Value when a test compound is added
      NSB: Non-specific Binding (an amount of non-specific binding)
      B₀ : Maximum binding amount

A compound obtained by using the present screening or method or screening kit, or a salt thereof, is a compound which has the activity of altering binding of a ligand with the present receptor protein and the like and, specifically, (a) a compound having the cell stimulating activity (for example, the activity promoting or inhibiting arachidonic acid release, acetylcholine release, intracellular Ca²⁺ release, intracellular cAMP production, intracellular cGMP production, inositol phosphate production, cell membrane potential fluctuation, intracellular protein phosphorylation, c-fos activation, and reduction of pH) via a G protein coupled receptor (so called agonist for the present receptor protein), (b) a compound having no cell stimulating activity (so called antagonist for the present receptorprotein), (c) a compound which enhances binding force of a ligand with the present G protein coupled receptor protein, or (d) a compound which decreases binding force of a ligand with the present G protein coupled receptor protein.

Examples of the compound include a peptide, a protein, a non-peptide compound, a synthetic compound and a fermentation product and these compounds may be a novel compound or the known compound.

Since an agonist for the present receptor protein and the like has the same activity as the physiological activity harbored by a ligand for the present receptor protein and the like, it is useful as a safe and low toxic medicine depending upon the ligand activity.

Since an antagonist for the present receptor protein and the like can suppress the physiological activity harbored by a ligand for the present receptor protein and the like, it is useful as a safe and low toxic medicine for suppressing the ligand activity.

A compound which enhances binding force of a ligand with the present G protein coupled receptor protein is useful as a safe and low toxic medicine for enhancing the physiological activity harbored by a ligand for the present receptor protein and the like.

A compound which decreases binding force of a ligand with the present G protein coupled receptor protein is useful as a safe and low toxic medicine for decreasing the physiological activity harbored by a ligand for the present receptor protein and the like.

When a compound obtained by using the present screening method or screening kit, a salt thereof is used as the aforementioned pharmaceutical composition, the use can be implemented according to the conventional means. For example, the compound can be formulated into tablets, capsules, elixirs, microcapsules, sterile solutions, and suspensions as in the aforementioned drug comprising the present receptor protein.

Since the thus obtained preparations are safe and low toxic, for example, they can be adiministered to a human being or a mammal (for example, rat, mouse, rabbit, sheep, pig, cow, cat, dog and monkey).

A dose of the compound or a salt thereof is different depending upon an administration subject, a subject organ, sympton and an administration method and, when orally administered, the dose is generally about 0.1 to 100 mg, preferably about 1.0 to 50 mg, more preferably about 1.0 to 20 mg per day, for example, in a hypertention patient (60 kg). When parenterally administered, one time dose is different depending upon an administration subject, a subject org an, sympton and administration method and it is advantageous to administer the compound by intravenous injection at an amount of around about 0.01 to 30 mg, preferably around about 0.1 to 20 mg, more preferably around about 0.1 to 10 mg in the form of an injectable, for example, in a hypertension patient (60 kg). In the case of other animals, an amount calculated per 60 kg can be administered.

### (8) An agent for preventing and/or treating various diseases comprising a compound which alters binding of the present G protein coupled receptor protein with a ligand (agonist and antagonist)

The present receptor protein is considered to play some important role in the living body such as the central function as described above. Therefore, a compound which alters binding of the present receptor protein with the ligand (agonist and antagonist) can be used as an agent for preventing and/or treating diseases associated with the deficient function of the present receptor protein.

When the compound is used as an agent for preventing and/or treating diseases associated with the deficient function of the present receptor protein, it can be formulated into preparations according to the conventional means.

For example, the compound can be used orally as tablets coated with a sugar-coating if necessary, capsules, elixirs and microcapsules, or parenterally used as an injection such as sterile solutions with water or other pharmaceutically acceptable solution, or suspensions. For examples, the preparations may be produced by blending the compound with the physiologically recognized known carriers, flavors, excipients, vehicles, antiseptics, stabilizers and binders in an unit dosage form required for generally recognized pharmacy. An amount of an ingredient in these preparations is such that a suitable volume in an indicated range can be obtained.

The additives which can be admixed with the tablets, capsules etc. include, for example, binders such as gelatin, corn starch, tragacanth, gum arabic, excipients such as crystalline cellulose, swelling agents such as corn starch, gelatin and alginic acid, lubricants such as magnesium stearate, sweeteners such as sucrose, lactose and saccharine, and flavors such as peppermint, akamono oil and cherry. When one capsule is in a unit form, liquid carriers such as fats and oils can be contained in the materials described above. The aseptic composition for injection can be formulated according to conventional pharmaceutical manufacturing by dissolving or suspending the active material and naturally occurring vegetable oils such as sesame oil and coconut oil in vehicles such as injection water. The aqueous solution for injection includes, for example, physiological saline or an isotonic solution containing glucose and other supplementary agents (e.g., D-sorbitol, D-mannitol, sodium chloride etc.), and may be used in combination with suitable solubilizer such as alcohols (e.g., ethanol etc.), polyalcohols (e.g., propylene glycol, polyethylene glycol etc.) and nonionic surfactants (e.g., Polysorbate 80™, HCO-50 etc.). The oily solution includes, for example, sesame oil, soybean oil etc., and may be used in combination with solubilizer such as benzyl benzoate, benzyl alcohol etc.

In addition, the above preventing or treating agent may be blended, for example, with the buffer (e.g., phosphatebuffer, sodium acetate buffer etc.), soothing agents (e.g., benzalkonium chloride, procaine hydrochloride etc.), stabilizers (e.g., human serum albumin, polyethylene glycol etc.), preservatives (e.g., benzyl alcohol, phenol etc.), antioxidants etc. Usually, the prepared injection is introduced into suitable ampoules.

Since the thus obtained preparations are safe and low toxic, for example, they can be administered to a human being or a mammal (for example, rat, mouse, rabbit, sheep, pig, cow, cat, dog and monkey).

A dose of the compound or a salt thereof is different depending upon an administration subject, a subject organ, sympton and an administration method and, orally administered, the dose is generally about 0.1 to 100 mg, preferably about 1.0 to 50 mg, more preferably about 1.0 to 20 mg per day, for example, in a hypertention patient (60 kg). When the compound is administered parenterally, one time dose is different depending upon an administration subject, a subject organ, sympton and an administration method and, for example, it is advantageous to administer the compound by intravenous injection at an amount of around about 0.01 to 30 mg, preferably around about 0.1 to 20 mg, more preferably around about 0.1 to 10 mg per day in the form of an injectable, for example, in a hypertension patient (60 kg). In the case of other animals, an amount calculated per 60 kg can be administered.

### (9) Quantitation of the present receptor protein or a partial peptide thereof or a salt thereof

Since the present antibody can specifically recognize the present receptor protein and the like, it can be used for quantitating the present receptor protein and the like in a test solution, in particular, for quantitation by a sandwich immunoassay. That is, the present invention provides, for example, (i) a method for quantitating the present receptor protein and the like in a test solution, which comprises competitively reacting the present antibody with a test solution and labeled receptor protein and the like, and measuring a proportion of the labeled receptor protein and the like bound to the antibody,
(ii) a method for quantitating the present receptor protein and the like in a test solution, which comprises reacting simultaneously or continuously a test solution with the present antibody insolubilized on a carrier and the labeled present antibody, and measuring the activity of a labeling agent on a insolubilized carrier.

In the above (ii), it is preferable that one antibody is an antibody which recognizes a N-terminal of the present receptor protein and the like, and the other antibody is an antibody which reacts with a C-terminal of the present receptor protein and the like.

By using a monoclonal antibody to the present receptor protein and the like (hereinafter referred to as present monoclonal antibody in some cases), measurement of the present receptor protein can be conducted and, additionally, detection with tissue staining can be conducted. For these objects, an antibody molecule itself may be used, or a F(ab')2 fraction, a Fab' fraction or a Fab fraction may be used. A method for measuring an antibody to the present receptor protein is not particularly limited but any measuring methods may be used as long as they are a method for measuring by detecting an amount of an antibody, an antigen or a antibody-antigen complex corresponding to an amount of an antigen in a test solution (for example, an amount of a receptor protein) by the chemical or physical means, and calculating this form a standard curve made using a standard solution comprising the known amount of an antigen. For example, nephrometry, a competition method, an immunometric method and a sandwich method are suitably used and, in respect of sensitivity and specificity, it is particularly preferable to use the sandwich method described later.

As a labeling agent used in a measuring method using a labeling substance, for example, a radioactive isotope, an enzyme, a fluorogenic substance and a chromogenic substance are used. As the radioactive isotope, for example, [¹²⁵I], [¹³¹I], [³H] and [¹⁴C] are used. As the above enzyme, enzymes which are safe and have the larger specific activity are preferable. For example, β-galactosidase, β-glucosidase, alkaline phosphatase, peroxidase and malic acid dehydrogenase are used. As the fluorogenic substance, for example, fluorescamine and fluorescein isothiocyanate are used. As the chromogenic substance, for example, luminol, luminol derivative, luciferin, and lucigenin are used. Further, biotin-avidin system may be used for binding an antibody or an antigen with a labeling agent.

Upon insolubilization of an antigen or an antibody, physical adsorption may be used. Alternatively, chemical binding usually used for insolubilizing or fixing proteins or enzymes may be used. As the carrier, for example, insoluble polysaccharides, such as agarose, dextran and cellulose, synthetic resins such as polystyrene, polyacrylamide and silicone, or glasses are used.

In the sandwich method, an amount of the present receptor protein in a test solution can be quantitated by reacting a test solution with the insolubilized present monoclonal antibody (primary reaction), reacting with the labeled present monoclonal antibody (secondary reaction), and measuring the activity of a labeling agent on an insolubilized carrier. The primary reaction and the secondary reaction may be conducted in the reverse order, or simultaneously or at different times. A labeling agent and a method for insolubilization may be according to those described above.

In an immunoassay via sandwich method, an antibody used for a solid phase antibody or a labeling antibody is not necessarily one kind but a mixture of two or more kinds of antibodies maybe used for the purpose of improving the measuring sensitivity.

In a method for measuring a receptor protein and the like by the present sandwich method, as the present monoclonal antibody used in the primary reaction and the secondary reaction, antibodies having different parts binding to the receptor protein and the like are preferably used. That is regarding antibodies used for the primary reaction and the secondary reaction, for example, when an antibody used for the secondary reaction recognizes a C-terminal of a receptor protein, as an antibody used in the primary reaction, an antibody which recognizes a part other than C-terminal, for example, a N-terminal is preferably used.

The present monoclonal antibody can be used, for example, in a nephrometry, competition method, an immunometric method or nephrometry. In the competition method, after an antigen in the test solution and a labeled antigen are competitively reacted with an antibody, an unreacted labeled antigen (F) and a labeled antigen bound to an antibody (B) are separated (B-F separation), an amount of either of labeled B or F is measured to quantitate an amount of an antigen in a test solution. In the present reaction method, a solution method using a soluble antibody as an antibody, using polyethylene glycol in B/F separation and using a solid phased antibody as the primary antibody, and a solid method using a solid phased antibody as the primary antibody, or using a soluble antibody as the primary antibody and a solid phased antibody as the secondary antibody, are used.

In the immunometric method, an antigen in a test solution and a solid phased antigen are competitively reacted with a constant amount of a labeled antibody and, thereafter, a solid phase and a solution phase are separated, or an antigen in a test solution and an excessive amount of a labeled antibody are reacted and a solid phased antigen is added thereto to bind an unreacted labeled antibody to a solid phase and, thereafter, a solid phase and a solution phase are separated.

In addition, in nephrometry, an amount of an insoluble precipitate produced as a result of an antigen-antibody reaction in a gel or a solution, is measured. Even when an amount of an antigen in a test solution is small and only a small amount of a precipitate is obtained, the laser nephrometry utilizing laser scattering is suitably used.

When these individual immunoassays are applied to the present invention, setting of the particular conditions and operations are not required. A system for measuring the present receptor protein or a salt thereof may be constructed by adding the normal technical consideration of a person skilled in the art to the normal conditions and operations in respective methods. For the details of these general technical means, one can see reviews and books [for example, see "Radioimmunoassay" ed. by Hiroshi Irie] (Kodansha, published by 1974), "Radioimmunoassay, a second series" ed. by Hiroshi Irie (Kodansha, published in 1979), "Enzyme immunoassay" ed. by Eigi Ishikawa (Igakushoin, published in 1978), "Enzyme immunoassay"(2nd edition) ed. by Eigi Ishikawa (Igakushoin, published in 1982), "Enzyme immunoassay" (3rd edition) ed. by Eigi Ishikawa (Igakushoin, published in 1987), (Methods in ENZYMONOLOGY) Vol.70 (Immunochemical Techniques (Part A)), ibid. Vol.73(Immunochemical Techniques(Part B), ibid. Vol.74 (Immunochemical Techniques (Part C), ibid. Vol.84 (Immunochemical Techniques (Part D: Selected Immunoassays)), ibid. Vol.92 (Immunochemical Techniques (Part E: Monoclonal Antibodies and General Immunoassay Methods)), ibid. Vol. 121 (Immunochemical Techniques (Part I: Hybridoma Technology and Monoclonal Antibodies)) (published by Academic Press)].

The present receptor protein or a salt thereof can be quantitated with better sensitivity by using the present antibody as described above.

Further, various diseases associated with the deficient functions of the present receptor protein can be diagnosed by quantitating the present receptor protein or a salt thereof in the living body by using the antibody.

In addition, the present antibody can be used for specifically detecting the present receptor protein and the like present in a specimen such as body fluid and tissues. In addition, the present antibody can be used for preparing an antibody column used for purifying the present receptor protein and the like, detecting the present receptor protein and the like in each fraction upon purification, and analyzing the behavior of the present receptor protein in a test cell.

### (10) A method for screening a compound which alters an amount of the present receptor protein or a partial peptide thereof in a cell membrane

Since the present antibody can recognize the present receptor protein or a partial peptide thereof or a salt thereof, it can be used for screening a compound which alters an amount of the present receptor protein or a partial peptide thereof in a cell membrane.

That is, the present invention provides for example:
(i) a method for screening a compound which alters an amount of the present receptor protein or a salt thereof in a cell membrane, by rupturing tissues or cell isolated from (a) blood, (b) particular organ, or (c) tissues or an organ of anon-human mammal, isolating a cell membrane fraction, and quantitating the present receptor protein or a partial peptide thereof contained in a cell membrane fraction,
(ii) a method for screening a compound which alters an amount of the present receptor protein or a partial peptide thereof in a cell membrane, by rupturing a transformant and the like expressing the present receptor protein or a partial peptide thereof, isolating a cell membrane fraction, and quantitating the present receptor protein or a partial peptide thereof contained in a cell membrane fraction,
(iii) a method for screening a compound which alters an amount of the present receptor protein or a partial peptide thereof in a cell membrane, by preparing tissues or cells isolated from (a) blood, (b) particular organ, or (c) an organ of a non-human mammal into sections, and quantitating a degree of staining of a receptor protein on a cell superficial layer by an immunostaining method to confirm the protein on a cell membrane,
(iv) a method for screening a compound which alters an amount of the present receptor protein or a partial peptide thereof, by preparing a transformant and the like expressing the present receptor protein or a partial peptide thereof into sections, and quantitating a degree of staining of the receptor protein on a cell superficial layer by an immunostaining method to confirm the preotein on a cell membrane.

Quantitation of the present receptor protein or a partial peptide thereof contained in a cell membrane fraction is conducted as follows:
(i) adrug (forexample, anti-dementia drug, blood pressure decreasing drug, anti-cancer agent and anti-obesity drug) or aphysical stress (for example, inundation stress, electric shock, light and dark, and low temperature) is given to a normal or disease model non-human mammal (for example, mouse, rat, rabbit, sheep, pig, cow, cat, dog and monkey and, more particularly, dementia rat, obesity mouse, arterial sclerosis rabbit and cancer carrying mouse) and, after a constant time has passed, isolated tissues or cells are obtained from blood, or particular organ (for example, brain, liver and kidney), or an organ. The resulting organs, tissues or cells are suspended in a suitable buffer (for example, Tris-hydrochloric acid buffer, phosphate buffer and Hepes buffer) , organs, tissues and cell are ruptured, and subjected to procedures such as centrifugation, filtration and column fractionation using surfactants (for example, Triton X100™ and Tween 20™) to obtain a cell membrane fraction.

A cell membrane fraction refers to a fraction which contains a large amount of a cell membrane obtained by the per se known method, after rupture of a cell. As a method for breaking a cell, there are a method for squeezing a cell with a Potter-Elvehgem type homogenizer, breakage with a Waring Blener and Poltron (manufactured by Kinematica), breakage by ultrasound, and breakage by jetting a cell through a thin nozzle while pressuring with a French press. For fractionating a cell membrane, a fractionating method by centrifugal force such as a fractionating centrifugation separating method and a density gradient centrifugation separating method is mainly used. For example, the broken cell solution is centrifuged at a low speed (500 rpm to 3000 rpm) for a shorter period of time (usually, about 1 minute to 10 minutes), the supernatant is further centrifuged at a high speed (15000 rpm to 30000 rpm) usually for 30 minutes to 2 hours, and the resulting precipitate is used as a membrane fraction. The expressed receptor protein and the like as well as phospholipid derived from a cell and a membrane component such as a membrane protein are contained at a large amount in the membrane fraction.

The present receptor protein or a salt thereof contained in a cell membrane fraction can be quantitated, for example, by a sandwich immunoassay using the present antibody or a Western blot analysis.

Such the sandwich immunoassay can be conducted as the aforementioned method, and Western blot can be conducted by the means known per se.
(ii) a transformant expressing the present receptor protein or a partial peptide thereof is prepared according to the aforementioned method, and the present receptor protein or a partial peptide thereof contained in a cell membrane fraction can be quantitated.

Screening of a compound which alters an amount of the present receptor protein or a partial peptide thereof in a cell membrane can be conducted by
(i) administering a test compound to a normal or a disease model non-human mammal a constant period of time before impartation of a drug or a physical stress (30 minutes before to 24 hours before, preferably 30 minutes before to 12 hours before, more preferably 1 hour before to 6 hours before), or a constant period of time after (30 minutes after to 3 days after, preferably 1 hour after to 2 days after, more preferably 1 hour after to 24 hours after), or simultaneously with a drug or a physical stress and, after a constant period of time has passed after administration (30 minutes after to 3 days after, preferably 1 hour after to 2 days after, more preferably 1 hour after to 24 hours after) , quantitating an amount of the present receptor protein or a partial peptide thereof in a cell membrane, and can be conducted by
(ii) mixing a test compound into a medium upon culturing of a transformant according to the conventional method and, after cultured for a constant period of time (1 day after to 7 days after, preferably 1 day after 3 cays after, more preferably 2 days after to 3 days after, quantitating an amount of the present receptor protein or a partial peptide thereof in a cell membrane.
   More particularly, confirmation of the present receptor protein or a partial peptide thereof contained in a cell membrane fraction can be conducted as follows:
(iii) a drug (for example, anti-dementia drug, blood pressure decreasing drug, anti-cancer agent and anti-obesity drug) or a physical stress (for example, inundation stress, electric shock, light and dark and low temperature) is given to a normal or disease model non-human mammal (for example, mouse, rat, rabbit, sheep, pig, cow, cat, dog and monkey and, more particularly, dementia rat, obesity mouse, arterial sclerosis rabbit and cancer carrying mouse) and, after a constant period of time has passed, tissues or cells isolated from blood, or particular organ (for example, brain, liver and kidney), or an organ are obtained. The resulting organ, tissues or cells are prepared into tissue sections according to the conventional method, and immunostaining is conducted using the present antibody. An amount of the present receptor protein or a partial peptide thereof in a cell membrane can be confirmed quantitatively or qualitatively by quantitating a degree of staining of the receptor protein on a cell superficial layer to confirm the protein on a cell membrane.
(iv) confirmation can be also conducted by the similar means using a transformant expressing the present receptor protein or a partial peptide thereof.

A compound obtained by the present screening method or a salt thereof is a compound which has the activity of altering an amount of the present receptor protein or a partial peptide thereof in a cell membrane and, more particularly, (a) a compound which enhances the cell stimulating activity (for example, the activity promoting or inhibiting arachidonic acid release, acetylcholine release, intracellular Ca²⁺ release, intracellular cAMP production, intracellular cGMP production, inositol phosphate production, cell membrane potential fluctuation, intracellular protein phosphorylation, c-fos activation, and reduction of pH) via a G protein coupled receptor by increasing an amount of the present receptor protein or a partial peptide thereof in a cell membrane, and (b) a compound which reduces the cell stimulating activity by decreasing an amount of the present receptor protein or a partial peptide thereof in a cell membrane.

Examples of the compound include a peptide, a protein, a non-peptide compound, a synthetic compound and fermentation product and these compounds may be a novel compound or the known compound.

A compound which enhances the cell stimulating activity is useful as a safe and low toxic medicine for enhancing the physiological activity of the present receptor protein.

A compound which reduces the cell stimulating activity is useful as a safe and low toxic medicine for decreasing the physiological activity of the present receptor protein and the like.

When a compound obtained by the screening method or a salt thereof is used as a pharmaceutical composition, the use thereof can be implemented according to the conventional means. For example, it can be formulated into tablets, capsules, elixirs, microcapsules, sterile solutions or suspensions as in the aforementioned drug comprising the present receptor protein.

Since the thus obtained preparations are safe and low toxic, for example, they can be administered to a human being or a mammal (for example, rat, mouse, rabbit, sheep, pig, cow, cat, dog and monkey).

A dose of the compound or a salt thereof is different depending upon an administration subject, a subject organ, sympton and an administration method and, when orally administered, the dose is generally about 0.1 to 100 mg, preferably about 1.0 to 50 mg, more preferably about 1.0 to 20 mg per day, for example, in a hypertension patient (60 kg). When parenterally administered, one time dose is different depending upon an administration subject, a subject organ, sympton and an administration method and, for example, it is advantageous to administer the compound by intravenous injection at an amount of around about 0.01 to 30 mg, preferably around about 0.1 to 20 mg, more preferably around about 0.1 to 10 mg per day in the form of an injectable, for example , in a hypertension patient (60 kg). In the case of other animals, an amount calculated per 60 kg can be administered.

### (11) An agent for preventing and/or treating various diseases comprising a compound which alters an amount of the present receptor protein or a partial peptide thereof in a cell membrane

The present receptor protein is considered to play some important role in the living body such as the central function as described above. Therefore, a compound which alters an amount of the present receptor protein or a partial peptide thereof in a cell membrane can be used as an agent for preventing and/or treating diseases associated with the deficient functions of the present receptor protein.

When the compound is used as an agent for preventing and/or treating diseases associated with the deficient functions of the present receptor protein, it can be formulated into preparations according to the conventional means.

For example, the compound can be used orally as tablets coated with a sugar-coating if necessary, capsules, elixirs or microcapsules, or parenterally as injections such as sterile solutions with water or other pharmaceutically acceptable solutions and suspensions. For example, preparations may be produced by blending the compound together with physiologically recognized known carriers, flavors, excipients, vehicles, antiseptics, stabilizers or binders in an unit dosage form required for the generally recognized pharmacy. An amount of an active ingredient in these preparations is such that a suitable volume in an indicated range can be obtained.

The additives which can be admixed with the tablets, capsules etc. include, for example, binders such as gelatin, corn starch, tragacanth, gum arabic, excipients such as crystalline cellulose, swelling agents such as corn starch, gelatin and alginic acid, lubricants such as magnesium stearate, sweeteners such as sucrose, lactose and saccharine, and flavors such as peppermint, akamono oil and cherry. When one capsule is in a unit form, liquid carriers such as fats and oils can be contained in the materials described above. The aseptic composition for injection can be formulated according to conventional pharmaceutical manufacturing by dissolving or suspending the active material and naturally occurring vegetable oils such as sesame oil and coconut oil in vehicles such as injection water. The aqueous solution for injection includes, for example, physiological saline or an isotonic solution containing glucose and other supplementary agents (e.g. D-sorbitol, D-mannitol, sodium chloride etc.), and may be used in combination with suitable solubilizer such as alcohols (e.g., ethanol etc.), polyalcohols (e.g., propylene glycol, polyethylene glycol etc.) and nonionic surfactants (e.g., Polysorbate 80™, HCO-50 etc.). The oily solution includes, for example, sesame oil, soybean oil etc., and may be used in combination with solubilizer such as benzyl benzoate, benzyl alcohol etc. Usually, the prepared injection is introduced into suitable ampoules.

Since the thus obtained preparations are safe and low toxic, for example, they can be administered to a human being or a mammal (for example, rat, mouse, rabbit, sheep, pig, cow, cat, dog and monkey).

A dose of the compound or a salt thereof is different depending upon an administration subject, a subject organ, sympton and an administration method and, when orally administered, the dose is generally about 0.1 to 100 mg, preferably about 1.0 to 50 mg, more preferably about 1.0 to 20 mg per day, for example, in a hypertension patient (60 kg). When administered parenterally, one time dose is different depending upon an administration subject, a subject organ, sympton and an administration method and, for example, it is advantageous to administer the compound by intravenous injection at an amount of around about 0.01 to 30 mg, preferably around about 0.1 to 20 mg, more preferably around about 0.1 to 10 mg in the form of an injectable, for example, in a hypertension patient (60 kg). In the case of other animals, an amount calculated per 60 kg can be administered.

### (12) Neutralization with an antibody to the present receptor protein, a partial peptide thereof or a salt thereof

Neutralizing activity of an antibody to the present receptor protein or a partial peptide thereof or a salt thereof, relative to the receptor protein and the like means the activity of inactivating the signal transmission functions associated with the receptor protein. Therefore, when the antibody has the neutralizing activity, the signal transmission associated with the receptor protein, for example, the cell stimulating activity (for example, the activity promoting or inhibiting arachidonic acid release, acetylcholine release, intracellular Ca²⁺ release, intracellular CAMP production, intracellular cGMP production, inositol phosphate production, cell membrane potential fluctuation, intracellular protein phosphorylation, c-fos activation, and reduction of pH) via the receptor protein can be inactivated. Therefore, the antibody can be used for preventing and/or treating diseases caused by excess expression of the receptor protein.

### (13) Production of an animal having a DNA encoding the present G protein coupled receptor protein

A transgenic animal expressing the present receptor protein and the like can be produced using the present DNA. Examples of the animal include a mammal (for example, rat, mouse, rabbit, sheep, pig, cow, cat, dog and monkey)(hereinafter, abbreviated as animal in some cases) and, in particular, a mouse and a rabbit are suitable.

When the present DNA is transferred into a subject animal, it is generally advantageous to use a gene the DNA as a gene construct bound to downstream of a promoter which can express can be expressed in an animal cell. For example, when the present DNA derived from a rabbit is transferred, a DNA transferred animal producing the present receptor protein and the like at a large amount can be produced by micro-injecting a gene construct bound to downstream of various promoters which can express the present DNA derived from an animal having the high homology therewith in an animal cell, into a rabbit fertilized egg. Although as this promoter, for example, ubiquitous expression promoters such as a virus-derived promoter and a metallothionein promoter can be used, preferably, the NGF gene promoter and the enolase gene promoter which are specifically expressed in a brain are used.

Transference of the present DNA at a fertilized egg cell stage is maintained such that it is present in all germ cells and somatic cells in a subject animal. The fact that the present receptor protein and the like is present in germ cells of the produced animal after DNA transference means that all offsprings of the produced animal have the present receptor protein and the like in all of their germ cells and somatic cells. Offsprings of this kind of animal which inheritate a gene have the present receptor protein and the like in all their germ cells and somatic cells.

The present DNA transferred animal can be rear-passaged under the normal rearing environment as the DNA harboring animal by confirming that a gene is stably retained by mating.

Further, a homozygote animal harboring a transferred gene in both homologous chromosomes is obtained by mating a female and a male of an animal harboring a DNA of interest, and a female and a male of this animal can be mated to propagation-passage so that all offsprings have the DNA.

Since an animal with the present DNA transferred highly expresses the present receptor protein and the like, it is useful as an animal for screening an agonist or an antagonist for the present receptor protein and the like.

The present DNA transferred animal can be also used as a cell source for tissue culturing. For example, the present receptor protein and the like can be analyzed by analyzing directly a DNA or a RNA in tissues in the present DNA transferred mouse, or analyzing tissues in which the present receptor protein expressed by a gene is present. Cells of tissues having the present receptor protein and the like is cultured by the standard tissue culturing techniques and, by using them, the functions of cells from tissues which are generally difficult to culture such as a brain and peripheral tissues can be studied. In addition, by using the cells, for example, selection of medicines which enhance the functions of various tissues is possible. In addition, when there is a highly expressing cell strain, the present receptor protein and the like can be isolated and purified therefrom.

When bases and amino acids are indicated by abbreviations, they are based on abbreviations by IUPAC-IUB Commission on Biochemical Nomenclature or the conventional abbreviations in the art and examples thereof are shown below. In addition, when an optical isomer can be present regarding an amino acid, a L amino acid is indicated unless expressly indicated.
- DNA :: deoxyribonucleic acid
- cDNA :: complementary deoxyribonucleic acid
- A :: adenine
- T :: thymine
- G :: guanine
- C :: cytosine
- RNA :: ribonucleic acid
- mRNA :: messenger ribonucleic acid
- dATP :: deoxyadenosine triphosphate
- dTTP :: deoxyathymidine triphosphate
- dGTP :: deoxyguanosine triphosphate
- dCTP :: deoxycytidine triphosphate
- ATP :: adenosine triphosphate
- EDTA :: ethylenediamine tetraacetic acid
- SDS :: sodium dodecylsulfate
- Gly :: glycine
- Ala :: alanine
- Val :: valine
- Leu :: leucine
- Ile :: isoleucine
- Ser :: serine
- Thr :: threonine
- Cys :: cysteine
- Met :: methionine
- Glu :: glutamic acid
- Asp :: aspartic acid
- Lys :: lysine
- Arg :: arginine
- His :: histidine
- Phe :: phenylalanine
- Tyr :: tyrosine
- Trp :: tryptophan
- Pro :: proline
- Asn :: asparagine
- Gln :: glutamine
- pGlu :: pyroglutamic acid
- Me :: methyl group
- Et :: ethyl group
- Bu :: butyl group
- Ph :: phenyl group
- TC :: thyazolidine-4(R)-carboxyamide group

In addition, substituents, protecting groups and reagents which are frequently used are described by the following symbols.
- Tos :: p-toluenesulfonyl
- CHO :: formyl
- Bzl :: benzyl
- Cl₂Bzl :: 2,6-dichlorobenzyl
- Bom :: benzyloxymethyl
- z :: benzyloxycarbonyl
- C1 - Z :: 2-chlorobenzyloxycarbonyl
- Br-Z :: 2-bromobenzyloxycarbonyl
- Boc :: t-butoxycarbonyl
- DNP :: dinitrophenol
- Trt :: trityl
- Bum :: t-butoxymethyl
- Fmoc :: N-9-fluorenylmethoxycarbonyl
- HOBt :: 1-hydroxybenztriazole
- HOOBt :: 3,4-dihydro-3-hydroxy-4-oxo-1,2,3-benzotriazine
- HONB :: 1-hydroxy-5-norbornene-2,3-dicarboxyimide
- DCC :: N,N'-dicyclohexylcarbodiimide

SEQ ID Nos. of Sequence Listing of the present specification indicate the following sequences:
[SEQ ID No:1] shows an amino acid sequence of the present rat peripheral brainstem-derived novel G protein coupled receptor protein rOT7T009C.
[SEQ ID No:2] shows an amino acid sequence of the present rat peripheral brainstem-derived novel G protein coupled receptor protein rOT7T009T.
[SEQ ID No:3] shows a base sequence of a cDNA encoding the present rat peripheral brainstem-derived novel G protein coupled receptor protein rOT7T009C having an amino acid sequence represented by SEQ ID No:1.
[SEQ ID No:4] shows a base sequence of a cDNA encoding the present rat peripheral brainstem-derived novel G protein coupled receptor protein rOT7T009T having an amino acid sequence represented by SEQ ID No:2.
[SEQ ID No:5] shows a base sequence of a primer 1 used for cloning a cDNA encoding the present rat peripheral brainstem-derived novel G protein coupled receptor protein rOT7T009C and rOT7T009T.
[SEQ ID No:6] shows a base sequence of a primer 2 used for cloning a cDNA encoding the present rat peripheral brainstem-derived novel G protein coupled receptor protein rOT7T009C and rOT7T009T.
[SEQ ID No:7] shows an amino acid sequence of the present rat peripheral brainstem-derived novel G protein coupled receptor protein hOT7T009.
[SEQ ID No:8] shows a base sequence of a cDNA encoding the present human fetus brain-derived novel G protein coupled receptor protein hOT7T009 having an amino acid sequence represented by SEQ ID No:7.
[SEQ ID No:9] shows a base sequence of a primer 3 used for cloning a cDNA encoding the present human fetus brain-derived novel G protein coupled receptor protein hOT7T009.
[SEQ ID No:10] shows a base sequence of a primer 4 used for cloning a cDNA encoding the present human fetus brain-derived novel G protein coupled receptor protein hOT7T009.
[SEQ ID No:11] shows a base sequence of a primer 5 used for cloning a cDNA encoding the present human fetus brain-derived novel G protein coupled receptor protein hOT7T009.
[SEQ ID No:12] shows a base sequence of a primer 6 used for cloning a cDNA encoding the present human fetus brain-derived novel G protein coupled receptor protein hOT7T009.

A transformant Escherichia coli DH10B/pAK-rOT009C obtained in Example 1 below has been deposited at National Institute of Bioscience and Human-Technology (NIBH), Agency of Industrial Science & Technology, Ministry of International Trade & Industry as an accession number FERM BP-6550 since October 19, 1998, and at Institute for Fermentation, Osaka, Japan (IFO) as an accession number IFO 16208 since October 1, 1998.

A transformant Escherichia coli DH10B/pAK-hOT009 obtained in Example 2 below has been deposited at. National. Institute of Bioscience and Human-Technology (NIBH), Agency of Industrial Science & Technology, Ministry of International Trade & Industry as an accession number FERM BP-6610 since December 21, 1998, and at Institute for Fermentation, Osaka, Japan (IFO) as an accession number IFO 16223 since December 7, 1998.

The following Examples illustrate the present invention inmore detail but do not limit the scope of the present invention. Genetic procedures using Escherichia coli were according to a method described in Molecular cloning.

### Example 1

Cloning of, and determination of a base sequence of a cDNA encoding a rat peripheral brainstem derived-G protein coupled receptor protein

A PCR reaction was conducted using a cDNA of a rat peripheral brainstem as a template and using 2 primers, primer 1 (SEQ ID No:5) and primer 2 (SEQ ID No:6). Regarding the composition of the reaction solution of the reaction, a 1/10 amount of the above cDNA was used as a template, a 1/50 amount of Advantage cDNA Polymerase Mix (CLONETECH), each 0.2 µM of a primer 1 (SEQ ID No:5) and a primer 2 (SEQ ID No:6), 200 µM dNTPs and a buffer affixed to an enzyme were added to an amount of a solution of 50 µl. In the PCR reaction, after (a) 94 °C for 2 minutes, (b) a cycle of 94 °C for 30 seconds, and 72 °C for 2 minutes was repeated three times, (c) a cycle of 94 °C for 30 seconds, and 68 °C for 2 minutes was repeated three times, (d) a cycle of 94 °C for 30 seconds, 64 °C for 30 seconds, and 68°C for 2 minutes was repeated 30 times, (e) finally an elongation reaction was conducted at 68 °C for 8 minutes. The reaction product of the PCR reaction was subcloned into the plasmid vector pCR2.1 (Invitrogen) according to the formulation of the TA cloning kit (Invitrogen). This was introduced into Escherichia coli DH5α, clones having a cDNA were selected in a LB agar medium comprising amplicillin, sequences of individual clones were analyzed and, as a result, a cDNA sequence encoding a novel G protein coupled receptor protein (SEQ ID Nos: 3 and 4) was obtained. A novel G protein coupled receptor protein comprising an amino acid sequence (SEQ ID Nos: 1 and 2) resulting from this cDNA was designated as rOT7T009 (a protein comprising SEQ ID No:1: rOT7T009C; a protein comprising SEQ ID No:2: rOT7T009T).

The plasmid pAK-rOT009C in which a cDNA (SEQ ID No:3) encoding the present rat peripheral brainstem-derived G protein coupled receptor protein rOT7T009C was subcloned was introduced Escherichia coli DH10B by the method known per se, to obtain a transformant:Escherichia coli DH10B/pAK-rOT009C.

### Example 2

Cloning of, and determination of a base sequence of a cDNA encoding a human fetus brain-derived novel G protein coupled receptor protein hOT7009C

A PCR reaction was conducted using a cDNA of a human fetus brain (CLONTECH) as a template and using 2 primers, primer 3 (SEQ ID No:9) and primer 4 (SEQ ID No:10). Regarding the composition of the reaction solution of the reaction, a 1/25 amount of the above cDNA was used as a template, a 1/50 amount of Advantage cDNA Polymerase Mix (CLONETECH), each 0.2 µM of a primer 3 (SEQ ID No:9) and a primer 4 (SEQ ID No:10), 200 µM dNTPs and a buffer affixed to an enzyme were added to an amount of a solution of 25 µl. In the PCR reaction, after (a) 94 °C for 2 minutes, (b) a cycle of 94 °C for 20 seconds, and 72 °C for 2 minutes was repeated three times, (c) a cycle of 94 °C for 20 seconds, 66 °C for 20 seconds and 68 °C for 2 minutes was repeated three times, (d) a cycle of 94 °C for 20 seconds, 62 °C for 20 seconds, and 68 °C for 2 minutes was repeated 32 times, (e) finally an elongation reaction was conducted at 68 °C for 7 minutes. The reaction product of the PCR reaction was subcloned into the plasmid vector pCR2.1 (Invitrogen) according to the formulation of the TA cloning kit (Invitrogen). This was introduced into Escherichia coli DH5α, clones having a cDNA were selected in a LB agar medium comprising amplicillin, sequences of individual clones were analyzed and, as a result, a cDNA sequence encoding a novel G protein coupled receptor protein (SEQ ID No:8) was obtained. A novel G protein coupled receptor protein comprising an amino acid sequence (SEQ ID No : 7) resulting from this cDNA was designated as hOT7T009.

The plasmid was purified from the above clone according to the method known per se, and a PCR reaction was conducted using that plasmid as a template and using 2 primers, primer 5 (SEQ ID No:11) and primer 6 (SEQ ID No:12). Regarding the composition of the reaction solution of the reaction, about 100 pg of the above plasmid was used as a template, a 1/50 amount of Advantage cDNA Polymerase Mix (CLONETECH), each 0.2 µM of a primer 3 (SEQ ID No:9) and a primer 4 (SEQ ID No:10), 200 µM dNTPs and a buffer affixed to an enzyme were added to an amount of a solution of 25 µl. In the PCR reaction, after (a) 94 °C for 2 minutes, (b) a cycle of 94 °C for 20 seconds, and 72 °C for 2 minutes was repeated three times, (c) a cycle of 94 °C for 20 seconds, and 68 °C for 2 minutes was repeated three times, (d) a cycle of 94 °C for 20 seconds, 64 °C for 20 seconds, and 68 °C for 2 minutes was repeated 25 times, (e) finally an elongation reaction was conducted at 68 °C for 7 minutes. The reaction product of the PCR reaction was subcloned into the plasmid vector pCR2.1 (Invitrogen) according to the formulation of the TA cloning kit (Invitrogen).

This was introduced into Escherichia coli DH5α according to the method known per se, to obtain a transformant:Escherichia coli DH5α/pCR2.1-hOT009. The plasmid pAK-hOT009 in which a cDNA (SEQ ID No:8) encoding the present human brain-derived novel G protein coupled receptor protein hOT7T009 excised from this plasmid with a restriction enzyme SalI/SpeI was subcloned, was introduced into Escherichia coli DH10B according to the method known per se, to obtain a transformant: Escherichia coli DH10B/pAK-hOT009.

### Example 3

### Establishment of CHO cell expressing rOT7T009

5×10⁵ CHO dhfr cells were seeded on a tissue culturing laboratory dish having a diameter of 10 cm, which was cultured for 24 hours. By using 10 µg of the rOT7T009C expression vector pAK-rOT009C obtained in Example 1 and using a gene transforming kit (FuGENE6 Trandfection Regeant, Boehringer Mannheim) according to a non-liposome method, a complex of a DNA:a reagent was formed. A medium was exchanged with a fresh one, and the DNA:reagent complex was added, which was incubated for 30 hours. Thereafter, cells in a laboratory dish was recovered with trypsin-EDTA treatment, which was recultured in a medium for selecting a transformant in the state of dilute cell density, to incarese a proportion of a transformant. Thereby, a clone of the cell strain CHO-rOT7T009C which highly expresses rOT7T009C stably was obtained.

After the whole RNA was extracted from the selected CHO cells expressing rOT7T009 according to the conventional method, an amount of a mRNA of rOT7T009C by a TaqMan method was measured and a copy number was calculated. Results are shwon in the following table.

**Table 1**

| Clone No. | Expression amount (copy/ng whole RNA) |
|---|---|
| 3 | 6335 |
| 5 | 4669 |
| 14 | 4678 |
| 18 | 5852 |
| 20 | 7602 |
| 21 | 3400 |
| 28 | 5500 |
| 29 | 4917 |

### Industrial applicability

The present G protein coupled receptor protein or a partial peptide thereof or a salt thereof and a polynucleotide (for example, DNA, RNA and its derivative) encoding the receptor protein or a partial peptide thereof can be used for (a) determination of a ligand (agonist), (b) obtaining of an antibody and anti-serum, (c) construction of expression system for a recombinant receptor protein, (d) development of a receptor binding assay system and screening of a drug candidate compound using the same expression system, (e) implementation of drug design based on comparison with a structurally analogous ligand or receptor, (f) reagent for making a probe and a PCR primer in gene diagnosis, (g) production of a transgenic animal, or medicine such as a gene preventing or treating agent.

## Claims

1. A G protein coupled receptor protein which comprises an amino acid sequence identical or substantially identical to an amino acid sequence represented by SEQ ID No : 1 or a salt thereof.

2. The G protein coupled receptor protein according to claim 1, wherein the amino acid sequence substantially identical to an amino acid sequence represented by SEQ ID No:1 is an amino acid sequence represented by SEQ ID No:2 or SEQ ID No:7 or a salt thereof.

3. A partial peptide of the G protein coupled receptor protein according to claim 1 or a salt thereof.

4. A polynucleotide which comprises a polynucleotide having a base sequence encoding the G protein coupled receptor protein according to claim 1.

5. The polynucleotide according to claim 4, which is a DNA.

6. The polynucleotide according to claim 4, which has a base sequence represented by SEQ ID No:3, SEQ ID No: 4 or SEQ ID No:8.

7. A recombinant vector which comprises the polynucleotide according to claim 4.

8. A transformant transformed with the recombinant vector according to claim 7.

9. A method for producing the G protein coupled receptor protein or a salt thereof, which comprises culturing a transformant according to claim 8 to produce the G protein coupled receptor protein according to claim 1.

10. An antibody to the G protein coupled receptor protein according to claim 1 or the partial peptide according to claim 3 or a salt thereof.

11. The antibody according to claim 10, which is a neutralizing antibody which inactivates signal transmission of the G protein coupled receptor protein according to claim 1.

12. A diagnostic agent which comprises the antibody according to claim 10.

13. A ligand for the G protein coupled receptor protein according to claim 1 or a salt thereof, which is obtainable by using the G protein coupled receptor protein according to claim 1 or the partial peptide according to claim 3 or a salt thereof.

14. A medicine which comprises the ligand for the G protein coupled receptor according to claim 13.

15. A method for determining a ligand for the G protein coupled receptor protein according to claim 1 or a salt thereof, which comprises using the G protein coupled receptor protein according to claim 1 or the partial peptide according to claim 3 or a salt thereof.

16. A method for screening a compound which alters binding of a ligand with the G protein coupled receptor protein according to claim 1, or a salt thereof, which comprises using the G protein coupled receptor protein according to claim 1 or the partial peptide according to claim 3 or a salt thereof.

17. A kit for screening a compound which alters binding of a ligand with the G protein coupled receptor protein or a salt thereof according to claim 1, or a salt thereof, which comprises the G protein coupled receptor protein according to claim 1 or the partial peptide according to claim 3 or a salt thereof.

18. A compound which alters binding of a ligand with the G protein coupled receptor protein or a salt thereof according to claim 1 or a salt thereof, which is obtainable by using the method for screening according to claim 16 or the kit for screening according to claim 17.

19. A medicine which comprises a compound which alters binding of a ligand with the G protein coupled receptor protein or a salt thereof according to claim 1, or a salt thereof, which is obtainable by the method for screening according to claim 16 or the kit for screening according to claim 17.

20. A polynucleotide which hybridizes with the polynucleotide according to claim 4 under the highly stringent conditions.

21. A polynucleotide which comprises a base sequence complementary to the polynucleotide according to claim 4 or a part thereof.

22. A method for quantitating a mRNA for the G protein coupled receptor protein according to claim 1, which comprises using the polynucleotide according to claim 4 or a part thereof.

23. A method for quantitating the G protein coupled receptor protein according to claim 1, which comprises using the antibody according to claim 10.

24. A method for diagnosing diseases associated with the function of the G protein coupled receptor according to claim 1, which comprises using the quantitating method according to claim 22 or claim 23.

25. A method for screening a compound which alters an amount of the G protein coupled receptor according to claim 1 to be expressed, or a salt thereof, which comprises using the method for quantitating according to claim 22.

26. A method for screening a compound which alters an amount of the G protein coupled receptor protein according to claim 1 or a salt thereof in a cell membrane, which comprises using the method for quantitating according to claim 23.

27. A compound which alters an amount of the G protein coupled receptor protein according to claim 1 or a salt thereof, which is obtainable by using the method for screening according to claim 25.

28. A compound which alters an amount of the G protein coupled receptor protein according to claim 1 or a salt thereof in a cell membrane, which is obtainable by using the method for screening according to claim 26.
